# EUROPEAN PATENT APPLICATION

(11) **EP 4 640 235 A1**
(43) Date of publication of application: **29.10.2025**
(21) Application number: 23907169.9
(22) Date of filing: 22.12.2023
(51) Int. Cl.: A61K 39/395, A61K 31/282, A61K 31/704, A61K 33/243, A61K 45/00, A61P 35/00, A61P 43/00

(54) **MEDICAMENT FOR TREATMENT AND/OR PREVENTION OF CANCER**

(30) Priority: 23.12.2022 JP 2022206138
(71) Applicant: Toray Industries, Inc., Tokyo 103-8666 (JP)
(72) Inventor: OKANO Fumiyoshi, Kamakura-shi, Kanagawa 248-8555 (JP); TACHIBANA Kazushi, Kamakura-shi, Kanagawa 248-8555 (JP)
(74) Representative: Mewburn Ellis LLP
(86) International application number: PCT/JP2023/046061
(87) International publication number: WO 2024/135809

(57) **Abstract**

Provided is a medicament for the treatment and/or prevention of cancer. A medicament for the treatment and/or prevention of cancer comprises: an antibody having immunological reactivity with the CAPRIN-1 protein or a fragment thereof; and an anthracycline-based drug and a platinum agent, or an anthracycline-based drug, a platinum agent, and an angiogenesis inhibitor, all together or in separate combinations (excluding pyrimidine-based drugs).

## Description

### Technical Field

The present invention relates to a medicament for treatment and/or prevention of a cancer, comprising: an antibody against CAPRIN-1 protein, or a fragment thereof; and an anthracycline-based drug and a platinum formulation, or an anthracycline-based drug, a platinum formulation, and an angiogenesis inhibitor.

### Background Art

Various antibody medicines targeting specific antigen proteins on cancer cells are applied as therapeutic agents for cancers with fewer side effects to cancer treatment because of their cancer specificity. For example, cytoplasmic-activation and proliferation-associated protein 1 (CAPRIN-1) is expressed on cell membrane surfaces of many solid cancers. Antibodies against this CAPRIN-1 protein are known to be promising in pharmaceutical uses for treatment and/or prevention of cancers (Patent Literature 1).

In recent years, treatment methods using combinations of pluralities of therapeutic agents for cancer have been clinically used as standard treatment methods in order to enhance the effectiveness of the therapeutic agents for cancers. In general, for example, colon cancer is treated by a treatment method using a combination of irinotecan, folinic acid, and fluorouracil; breast cancer is treated by a treatment method using a combination of doxorubicin and cyclophosphamide or a combination of paclitaxel, trastuzumab, and pertuzumab; and gastric cancer is treated by a treatment method using a combination of anticancer agents such as carboplatin and fluorouracil. Therapeutic agents for cancers comprising anti-CAPRIN-1 antibodies as active ingredients have also been confirmed to have therapeutic effects on cancers by combinations with chemotherapeutics (Patent Literature 2). However, treatment of a cancer by a combination of chemotherapeutics is not effective for every cancer to which the treatment is applied, and few combinations of chemotherapeutics synergistically drastically enhance therapeutic effects, though some combinations additively enhance therapeutic effects.

Ovarian cancer is likely to metastasize into the abdominal cavity, the lymph node, the liver or the lung. In particular ovarian cancers that have progressed in Stage III or Stage IV, more than 50% of the cases recur within two years after the initial treatment, and thus, the cancer has an extremely high malignancy. For such a metastatic cancer primary to an ovarian cancer, a therapy with a combination of gemcitabine and carboplatin (GC) is used. In the case of a patient whose PFI (Platinum-Free Interval), interval from the termination of the initial chemotherapy to a recurrence, is six months or more (what is called a platinum-sensitive patient), the response rate of the treatment after the recurrence is 40% or more, but in the case of a patient whose PFI, interval from the termination of the initial chemotherapy to a recurrence, is less than six months (what is called a platinum-insensitive patient), the response rate of the treatment after the recurrence is 10% or less, which means an extremely poor prognosis (Non Patent Literature 1). As an intensification therapy for a platinum-sensitive ovarian cancer patient, a therapy with a combination of the three agents, namely, bevacizumab in addition to gemcitabine and carboplatin (GC-BEV) has been approved. As a result in a clinical trial, OCEANS, with a combination of the three agents, GC-BEV significantly extended the PFS (Progression-Free Survival), compared with GC, but made no significant difference in the median value of the OS (overall survival) (Non Patent Literature 2).

In recent years, chemotherapy with a combination of gemcitabine/carboplatin/bevacizumab or doxorubicin-encapsulated liposome formulation (PLD)/carboplatin/bevacizumab has been attempted for a patient with platinum-sensitive recurrent ovarian cancer, which is one of the ovarian cancers, with the expectation of improved therapeutic effects (Non Patent Literature 3). According to Non Patent Literature 3, in a gemcitabine/carboplatin/bevacizumab therapy for a patient with platinum-sensitive recurrent ovarian cancer, the progression-free survival PFS (median) was 11.6 months, and overall survival OS (median) is 27.8 months. On the other hand, in a doxorubicin encapsulated liposome formulation (PLD)/carboplatin/bevacizumab therapy, the progression-free survival PFS (median) is 13.3 months, the overall survival OS (median) is 31.9 months. Thus, it has been reported that the progression-free survival and the overall survival are slightly longer.

### Citation List

### Patent Literature

Patent Literature 1: WO2010/016526
Patent Literature 2: WO2011/096535

### Non Patent Literature

Non Patent Literature 1: Crit Rev Oncol Hematol, 2007, 64, 129-138
Non Patent Literature 2: J Clin Oncol, 2012, 30, 2039-2045
Non Patent Literature 3: The LANCET Oncol, 2020, 21 (5), 699-709

### Summary of Invention

### Technical Problem

An object of the present invention is to provide a medicament for treatment and/or prevention of a cancer specifically expressing CAPRIN-1 protein on a cell surface.

### Solution to Problem

As the result of intensive studies, the present inventors have found that a combined use (a combination) of three agents, namely, an antibody against CAPRIN-1 protein, or a fragment thereof, having an immunological reactivity with cancer cells, an anthracycline-based drug (for example, a doxorubicin hydrochloride liposome formulation (PLD)), and a platinum formulation (for example, carboplatin), or a combination of four agents, namely, an angiogenesis inhibitor (for example, bevacizumab) in addition to the three agents exerts a remarkably stronger antitumor effect in a treatment than a combination of three agents, namely, an anthracycline-based drug (for example, a doxorubicin hydrochloride liposome formulation (PLD)), a platinum formulation (for example, carboplatin), and an angiogenesis inhibitor (for example, bevacizumab). On the basis of these findings, the present invention has been completed.

Specifically, the present invention relates to the following embodiments (1) to (20).
(1) A medicament for treatment and/or prevention of cancer, comprising: an antibody or a fragment thereof having an immunological reactivity with CAPRIN-1 protein; an anthracycline-based drug; and a platinum formulation, together or separately in combination (excluding a pyrimidine-based drug).
(2) The medicament according to (1), further comprising an angiogenesis inhibitor together or separately in combination.
(3) The medicament according to (1) or (2), wherein the anthracycline-based drug is doxorubicin or a doxorubicin hydrochloride liposome formulation (PLD).
(4) The medicament according to any of (1) to (3), wherein the platinum formulation is carboplatin, cisplatin, oxaliplatin, nedaplatin, and/or miriplatin.
(5) The medicament according to any of (2) to (4), wherein the angiogenesis inhibitor is bevacizumab.
(6) The medicament according to any of (1) to (5), wherein the antibody or the fragment thereof has an immunological reactivity with CAPRIN-1 protein having an amino acid sequence shown by any one of the even numbered SEQ ID NOs among SEQ ID NOs: 2 to 30, or an amino acid sequence having 80% or more sequence identity with the amino acid sequence.
(7) The medicament according to any of (1) to (6), wherein the antibody or the fragment thereof has an immunological reactivity with an extracellular region of CAPRIN-1 protein present on a cancer cell surface.
(8) The medicament according to any of (1) to (7), wherein the antibody or the fragment thereof has an immunological reactivity with a partial polypeptide of CAPRIN-1 protein, the partial polypeptide having an amino acid sequence represented by any one of SEQ ID NOs: 31 to 35, 296 to 299, 308 and 309, or an amino acid sequence having 80% or more sequence identity with the amino acid sequence.
(9) The medicament according to any of (1) to (8), wherein the antibody is a monoclonal antibody or a polyclonal antibody.
(10) The medicament according to any of (1) to (9), wherein the antibody or a fragment thereof is any one of the following (A) to (M):
   (A) an antibody or a fragment thereof comprising a heavy-chain variable region comprising complementarity determining regions of SEQ ID NOs: 36, 37 and 38 (CDR1, CDR2 and CDR3, respectively) and a light-chain variable region comprising complementarity determining regions of SEQ ID NOs: 40, 41 and 42 (CDR1, CDR2 and CDR3, respectively), and having an immunological reactivity with CAPRIN-1 protein;
   (B) an antibody or a fragment thereof comprising a heavy-chain variable region comprising complementarity determining regions of SEQ ID NOs: 44, 45 and 46 (CDR1, CDR2 and CDR3, respectively) and a light-chain variable region comprising complementarity determining regions of SEQ ID NOs: 48, 49 and 50 (CDR1, CDR2 and CDR3, respectively), and having an immunological reactivity with CAPRIN-1 protein;
   (C) an antibody or a fragment thereof comprising a heavy-chain variable region comprising complementarity determining regions of SEQ ID NOs: 52, 53 and 54 (CDR1, CDR2 and CDR3, respectively) and a light-chain variable region comprising complementarity determining regions of SEQ ID NOs: 56, 57 and 58 (CDR1, CDR2 and CDR3, respectively), and having an immunological reactivity with CAPRIN-1 protein;
   (D) an antibody or a fragment thereof comprising a heavy-chain variable region comprising complementarity determining regions of SEQ ID NOs: 60, 61 and 62 (CDR1, CDR2 and CDR3, respectively) and a light-chain variable region comprising complementarity determining regions of SEQ ID NOs: 64, 65 and 66 (CDR1, CDR2 and CDR3, respectively), and having an immunological reactivity with CAPRIN-1 protein;
   (E) an antibody or a fragment thereof comprising a heavy-chain variable region comprising complementarity determining regions of SEQ ID NOs: 170, 171 and 172 (CDR1, CDR2 and CDR3, respectively) and a light-chain variable region comprising complementarity determining regions of SEQ ID NOs: 173, 174 and 175 (CDR1, CDR2 and CDR3, respectively), and having an immunological reactivity with CAPRIN-1 protein;
   (F) an antibody or a fragment thereof comprising a heavy-chain variable region comprising complementarity determining regions of SEQ ID NOs: 176, 177 and 178 (CDR1, CDR2 and CDR3, respectively) and a light-chain variable region comprising complementarity determining regions of SEQ ID NOs: 179, 180 and 181 (CDR1, CDR2 and CDR3, respectively), and having an immunological reactivity with CAPRIN-1 protein;
   (G) an antibody or a fragment thereof comprising a heavy-chain variable region comprising complementarity determining regions of SEQ ID NOs: 182, 183 and 184 (CDR1, CDR2 and CDR3, respectively) and a light-chain variable region comprising complementarity determining regions of SEQ ID NOs: 185, 186 and 187 (CDR1, CDR2 and CDR3, respectively), and having an immunological reactivity with CAPRIN-1 protein;
   (H) an antibody or a fragment thereof comprising a heavy-chain variable region comprising complementarity determining regions of SEQ ID NOs: 188, 189 and 190 (CDR1, CDR2 and CDR3, respectively) and a light-chain variable region comprising complementarity determining regions of SEQ ID NOs: 191, 192 and 193 (CDR1, CDR2 and CDR3, respectively), and having an immunological reactivity with CAPRIN-1 protein;
   (I) an antibody or a fragment thereof comprising a heavy-chain variable region comprising complementarity determining regions of SEQ ID NOs: 146, 147 and 148 (CDR1, CDR2 and CDR3, respectively) and a light-chain variable region comprising complementarity determining regions of SEQ ID NOs: 149, 150 and 151 (CDR1, CDR2 and CDR3, respectively), and having an immunological reactivity with CAPRIN-1 protein;
   (J) an antibody or a fragment thereof comprising a heavy-chain variable region comprising complementarity determining regions of SEQ ID NOs: 272, 273 and 274 (CDR1, CDR2 and CDR3, respectively) and a light-chain variable region comprising complementarity determining regions of SEQ ID NOs: 275, 276 and 277 (CDR1, CDR2 and CDR3, respectively), and having an immunological reactivity with CAPRIN-1 protein;
   (K) an antibody or a fragment thereof comprising a heavy-chain variable region comprising complementarity determining regions of SEQ ID NOs: 290, 291 and 292 (CDR1, CDR2 and CDR3, respectively) and a light-chain variable region comprising complementarity determining regions of SEQ ID NOs: 293, 294 and 295 (CDR1, CDR2 and CDR3, respectively), and having an immunological reactivity with CAPRIN-1 protein;
   (L) an antibody or a fragment thereof comprising a heavy-chain variable region comprising complementarity determining regions of SEQ ID NOs: 300, 301 and 302 (CDR1, CDR2 and CDR3, respectively) and a light-chain variable region comprising complementarity determining regions of SEQ ID NOs: 304, 305 and 306 (CDR1, CDR2 and CDR3, respectively), and having an immunological reactivity with CAPRIN-1 protein; and
   (M) an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein.
(11) The medicament according to any of (1) to (10), wherein the antibody or the fragment thereof is any one of the following (a) to (al):
   (a) an antibody or a fragment thereof comprising a heavy-chain variable region comprising the amino acid sequence of SEQ ID NO: 39 and a light-chain variable region comprising the amino acid sequence of SEQ ID NO: 43;
   (b) an antibody or a fragment thereof comprising a heavy-chain variable region comprising the amino acid sequence of SEQ ID NO: 47 and a light-chain variable region comprising the amino acid sequence of SEQ ID NO: 51;
   (c) an antibody or a fragment thereof comprising a heavy-chain variable region comprising the amino acid sequence of SEQ ID NO: 55 and a light-chain variable region comprising the amino acid sequence of SEQ ID NO: 59;
   (d) an antibody or a fragment thereof comprising a heavy-chain variable region comprising the amino acid sequence of SEQ ID NO: 63 and a light-chain variable region comprising the amino acid sequence of SEQ ID NO: 67;
   (e) an antibody or a fragment thereof comprising a heavy-chain variable region comprising the amino acid sequence of SEQ ID NO: 68 and a light-chain variable region comprising the amino acid sequence of SEQ ID NO: 69;
   (f) an antibody or a fragment thereof comprising a heavy-chain variable region comprising the amino acid sequence of SEQ ID NO: 70 and a light-chain variable region comprising the amino acid sequence of SEQ ID NO: 71;
   (g) an antibody or a fragment thereof comprising a heavy-chain variable region comprising the amino acid sequence of SEQ ID NO: 72 and a light-chain variable region comprising the amino acid sequence of SEQ ID NO: 73;
   (h) an antibody or a fragment thereof comprising a heavy-chain variable region comprising the amino acid sequence of SEQ ID NO: 74 and a light-chain variable region comprising the amino acid sequence of SEQ ID NO: 75;
   (i) an antibody or a fragment thereof comprising a heavy-chain variable region comprising the amino acid sequence of SEQ ID NO: 76 and a light-chain variable region comprising the amino acid sequence of SEQ ID NO: 77;
   (j) an antibody or a fragment thereof comprising a heavy-chain variable region comprising the amino acid sequence of SEQ ID NO: 78 and a light-chain variable region comprising the amino acid sequence of SEQ ID NO: 79;
   (k) an antibody or a fragment thereof comprising a heavy-chain variable region comprising the amino acid sequence of SEQ ID NO: 80 and a light-chain variable region comprising the amino acid sequence of SEQ ID NO: 81;
   (l) an antibody or a fragment thereof comprising a heavy-chain variable region comprising the amino acid sequence of SEQ ID NO: 82 and a light-chain variable region comprising the amino acid sequence of SEQ ID NO: 83;
   (m) an antibody or a fragment thereof comprising a heavy-chain variable region comprising the amino acid sequence of SEQ ID NO: 84 and a light-chain variable region comprising the amino acid sequence of SEQ ID NO: 85;
   (n) an antibody or a fragment thereof comprising a heavy-chain variable region comprising the amino acid sequence of SEQ ID NO: 86 and a light-chain variable region comprising the amino acid sequence of SEQ ID NO: 87;
   (o) an antibody or a fragment thereof comprising a heavy-chain variable region comprising the amino acid sequence of SEQ ID NO: 88 and a light-chain variable region comprising the amino acid sequence of SEQ ID NO: 89;
   (p) an antibody or a fragment thereof comprising a heavy-chain variable region comprising the amino acid sequence of SEQ ID NO: 90 and a light-chain variable region comprising the amino acid sequence of SEQ ID NO: 91;
   (q) an antibody or a fragment thereof comprising a heavy-chain variable region comprising the amino acid sequence of SEQ ID NO: 92 and a light-chain variable region comprising the amino acid sequence of SEQ ID NO: 93;
   (r) an antibody or a fragment thereof comprising a heavy-chain variable region comprising the amino acid sequence of SEQ ID NO: 94 and a light-chain variable region comprising the amino acid sequence of SEQ ID NO: 95;
   (s) an antibody or a fragment thereof comprising a heavy-chain variable region comprising the amino acid sequence of SEQ ID NO: 96 and a light-chain variable region comprising the amino acid sequence of SEQ ID NO: 97;
   (t) an antibody or a fragment thereof comprising a heavy-chain variable region comprising the amino acid sequence of SEQ ID NO: 98 and a light-chain variable region comprising the amino acid sequence of SEQ ID NO: 99;
   (u) an antibody or a fragment thereof comprising a heavy-chain variable region comprising the amino acid sequence of SEQ ID NO: 100 and a light-chain variable region comprising the amino acid sequence of SEQ ID NO: 101;
   (v) an antibody or a fragment thereof comprising a heavy-chain variable region comprising the amino acid sequence of SEQ ID NO: 102 and a light-chain variable region comprising the amino acid sequence of SEQ ID NO: 103;
   (w) an antibody or a fragment thereof comprising a heavy-chain variable region comprising the amino acid sequence of SEQ ID NO: 104 and a light-chain variable region comprising the amino acid sequence of SEQ ID NO: 105;
   (x) an antibody or a fragment thereof comprising a heavy-chain variable region comprising the amino acid sequence of SEQ ID NO: 106 and a light-chain variable region comprising the amino acid sequence of SEQ ID NO: 107;
   (y) an antibody or a fragment thereof comprising a heavy-chain variable region comprising the amino acid sequence of SEQ ID NO: 108 and a light-chain variable region comprising the amino acid sequence of SEQ ID NO: 109;
   (z) an antibody or a fragment thereof comprising a heavy-chain variable region comprising the amino acid sequence of SEQ ID NO: 110 and a light-chain variable region comprising the amino acid sequence of SEQ ID NO: 111;
   (aa) an antibody or a fragment thereof comprising a heavy-chain variable region comprising the amino acid sequence of SEQ ID NO: 112 and a light-chain variable region comprising the amino acid sequence of SEQ ID NO: 113;
   (ab) an antibody or a fragment thereof comprising a heavy-chain variable region comprising the amino acid sequence of SEQ ID NO: 114 and a light-chain variable region comprising the amino acid sequence of SEQ ID NO: 115;
   (ac) an antibody or a fragment thereof comprising a heavy-chain variable region comprising the amino acid sequence of SEQ ID NO: 116 and a light-chain variable region comprising the amino acid sequence of SEQ ID NO: 117;
   (ad) an antibody or a fragment thereof comprising a heavy-chain variable region comprising the amino acid sequence of SEQ ID NO: 118 and a light-chain variable region comprising the amino acid sequence of SEQ ID NO: 119;
   (ae) an antibody or a fragment thereof comprising a heavy-chain variable region comprising the amino acid sequence of SEQ ID NO: 120 and a light-chain variable region comprising the amino acid sequence of SEQ ID NO: 121;
   (af) an antibody or a fragment thereof comprising a heavy-chain variable region comprising the amino acid sequence of SEQ ID NO: 122 and a light-chain variable region comprising the amino acid sequence of SEQ ID NO: 123;
   (ag) an antibody or a fragment thereof comprising a heavy-chain variable region comprising the amino acid sequence of SEQ ID NO: 124 and a light-chain variable region comprising the amino acid sequence of SEQ ID NO: 125;
   (ah) an antibody or a fragment thereof comprising a heavy-chain variable region comprising the amino acid sequence of SEQ ID NO: 126 and a light-chain variable region comprising the amino acid sequence of SEQ ID NO: 127;
   (ai) an antibody or a fragment thereof comprising a heavy-chain variable region comprising the amino acid sequence of SEQ ID NO: 128 and a light-chain variable region comprising the amino acid sequence of SEQ ID NO: 129;
   (aj) an antibody or a fragment thereof comprising a heavy-chain variable region comprising the amino acid sequence of SEQ ID NO: 130 and a light-chain variable region comprising the amino acid sequence of SEQ ID NO: 131;
   (ak) an antibody or a fragment thereof comprising a heavy-chain variable region comprising the amino acid sequence of SEQ ID NO: 132 and a light-chain variable region comprising the amino acid sequence of SEQ ID NO: 133; and
   (al) an antibody or a fragment thereof comprising a heavy-chain variable region comprising the amino acid sequence of SEQ ID NO: 303 and a light-chain variable region comprising the amino acid sequence of SEQ ID NO: 307.
(12) The medicament according to any of (1) to (11), wherein the antibody is a human antibody, a humanized antibody, a chimeric antibody or a single chain antibody.
(13) The medicament according to any of (1) to (12), wherein the cancer is a cancer expressing CAPRIN-1 protein on a cell membrane surface.
(14) The medicament according to any of (1) to (13), wherein the cancer is ovarian cancer, bile duct cancer, breast cancer, kidney cancer, pancreatic cancer, colon cancer, melanoma, lung cancer, renal cell carcinoma, Hodgkin's lymphoma, head and neck cancer, gastric cancer, mesothelioma, colorectal cancer, esophageal cancer, gastroesophageal junction cancer, hepatocellular carcinoma, glioblastoma, urothelial carcinoma, urinary bladder cancer, uterus cancer, primary central nervous system lymphoma, primary testicular lymphoma, biliary tract cancer, brain tumor, prostate cancer, leukemia, lymphoma, liver cancer, sarcoma, fibrosarcoma, mastocytoma, adrenocortical carcinoma, Ewing's tumor, multiple myeloma, testicular cancer, thyroid cancer, basal cell carcinoma, Paget's disease, or skin cancer.
(15) A drug efficacy increasing agent of a medicament for treatment and/or prevention of cancer, wherein the agent comprises, as active ingredients, an anthracycline-based drug and a platinum formulation (but not comprising a pyrimidine-based drug as an active ingredient), wherein the medicament comprises, as an active ingredient, an antibody or a fragment thereof having an immunological reactivity with CAPRIN-1 protein.
(16) The drug efficacy increasing agent according to (15), further comprising, as an active ingredient, an angiogenesis inhibitor.
(17) A drug efficacy increasing agent of a medicament for treatment and/or prevention of cancer, wherein the agent comprises, as an active ingredient, an antibody or a fragment thereof having an immunological reactivity with CAPRIN-1 protein, wherein the medicament comprises, as active ingredients, an anthracycline-based drug and a platinum formulation (but not comprising a pyrimidine-based drug as an active ingredient).
(18) The drug efficacy increasing agent according to (17), wherein the medicament further comprises, as an active ingredient, an angiogenesis inhibitor.
(19) A method for treating and/or preventing cancer, comprising administering: an antibody or a fragment thereof having an immunological reactivity with CAPRIN-1 protein; an anthracycline-based drug; and a platinum formulation, together or separately to a subject (but not administering a pyrimidine-based drug).
(20) The method according to (19), comprising further administering an angiogenesis inhibitor together or separately to a subject.

The present specification encompasses the contents disclosed in Japanese Patent Application No. 2022-206138, which is the basis of the priority of the present application.

### Advantageous Effects of Invention

The combined use of an antibody against CAPRIN-1 protein, or a fragment thereof, with an anthracycline-based drug and a platinum formulation, or an anthracycline-based drug, a platinum formulation, and an angiogenesis inhibitor, according to the present invention, exerts a stronger antitumor effect than an antibody against CAPRIN-1 protein, or a fragment thereof alone, and a combination of an anthracycline-based drug and a platinum formulation, or a combination of an anthracycline-based drug, a platinum formulation, and an angiogenesis inhibitor. Accordingly, a combined use of an antibody against CAPRIN-1 protein, or a fragment thereof, with an anthracycline-based drug and a platinum formulation, or an anthracycline-based drug, a platinum formulation, and an angiogenesis inhibitor is effective for treatment and/or prevention of cancer.

### Description of Embodiments

The antitumor activity of the combined use of an antibody against CAPRIN-1 protein or a fragment thereof (hereinafter, generically referred to as an "anti-CAPRIN-1 antibody") with an anthracycline-based drug and a platinum formulation or an anthracycline-based drug, a platinum formulation, and an angiogenesis inhibitor used in the present invention, can be evaluated by examining *in vivo* the inhibition of tumor growth in a tumor-bearing animal as mentioned later.

As used herein, the term "comprising together or separately in combination" means comprising a plurality of drugs in the form in which the drugs can be administered simultaneously or separately to a patient. The form may be, for example, in the form of what is called a mixed formulation in which a plurality of drugs are mixed, or may be in the form of what is called a kit formulation comprising a plurality of drugs as individual formulations.

Such a kit formulation according to the present invention may be, for example, a kit formulation comprising: a formulation (or pharmaceutical composition) containing an anti-CAPRIN-1 antibody; and a formulation (or pharmaceutical composition) containing an anthracycline-based drug and a platinum formulation or an anthracycline-based drug, a platinum formulation, and an angiogenesis inhibitor. Furthermore, a kit formulation according to the present invention may comprise another formulation (another known antitumor agent, excluding a pyrimidine-based drug) in addition to an anti-CAPRIN-1 antibody, an anthracycline-based drug and a platinum formulation, or an anthracycline-based drug, a platinum formulation and an angiogenesis inhibitor.

The term "combined use" or "in combination" described herein refers to simultaneous administration or administration in a predetermined interval of the anti-CAPRIN-1 antibody and of the anthracycline-based drug and the platinum formulation, or the anthracycline-based drug, the platinum formulation, and the angiogenesis inhibitor, as independent active ingredients to the same organism. The interval may be simultaneous administration or may be 30 minutes later, 1 hour later, 3 hours later, 6 hours later, 12 hours later, 1 day later, 3 days later, 5 days later, 7 days later, 2 weeks later, 3 weeks later, or 4 weeks later. Any one of an anti-CAPRIN-1 antibody; and a drug comprising an anthracycline-based drug and a platinum formulation, or an anthracycline-based drug, a platinum formulation, and an angiogenesis inhibitor may be administered, when the antibody or the drug, which has been administered in advance, exhibits its activity *in vivo.* The anti-CAPRIN-1 antibody may be administered first, or the drug comprising an anthracycline-based drug and a platinum formulation, or an anthracycline-based drug, a platinum formulation, and an angiogenesis inhibitor may be administered first.

The anti-CAPRIN-1 antibody according to the present invention may be a monoclonal antibody or a polyclonal antibody and is preferably a monoclonal antibody. The antibody of the present invention may be any type of antibody, as long as it can exhibit antitumor activity. The antibody is a recombinant antibody, a human antibody, a humanized antibody, a chimeric antibody, or a non-human animal antibody.

Subjects in need of treatment and/or prevention of cancer according to the present invention are mammals such as human, pet animals, livestock animals, or sport animals. The preferred subject is a human.

Medicaments for treatment and/or prevention of cancer, wherein the medicaments comprise, as active ingredients, an anti-CAPRIN-1 antibody, and an anthracycline-based drug and a platinum formulation, or an anthracycline-based drug, a platinum formulation, and an angiogenesis inhibitor (excluding a pyrimidine-based drug), and methods for treating and/or preventing cancers, related to the present invention, will be explained below.

### <Anti-CAPRIN-1 antibody>

Among CAPRIN-1 proteins having an amino acid sequence shown by any one of the even numbered SEQ ID NOs among SEQ ID NOs: 2 to 30, which are specific examples of antigens having an immunological reactivity with the anti-CAPRIN-1 antibody used in the present invention, the amino acid sequences shown by SEQ ID NOs: 6, 8, 10, 12 and 14 are amino acid sequences of canine CAPRIN-1 proteins; the amino acid sequences shown by SEQ ID NOs: 2 and 4 are amino acid sequences of human CAPRIN-1 proteins; the amino acid sequence shown by SEQ ID NO: 16 is an amino acid sequence of a bovine CAPRIN-1 protein; the amino acid sequence shown by SEQ ID NO: 18 is an amino acid sequence of an equine CAPRIN-1 protein; the amino acid sequences shown by SEQ ID NOs: 20, 22, 24, 26, and 28 are amino acid sequences of mouse CAPRIN-1 proteins; and the amino acid sequence shown by SEQ ID NO: 30 is an amino acid sequence of a chicken CAPRIN-1 protein.

The anti-CAPRIN-1 antibody used in the present invention may have an immunological reactivity with CAPRIN-1 protein variant having 80% or more, preferably 90% or more, more preferably 95% or more, and further preferably 99% or more sequence identity to the amino acid sequence shown by any one of the even numbered SEQ ID NOs among SEQ ID NOs: 2 to 30. The term "% sequence identity" as used herein means a percentage (%) of the number of identical amino acids (or nucleotides) to the total number of amino acids (or nucleotides) in the case that two sequences are aligned such that maximum similarity can be achieved with or without introduction of gaps.

In the present invention, the anti-CAPRIN-1 antibody refers to an antibody or a fragment thereof having an immunological reactivity with a full-length CAPRIN-1 protein or a fragment thereof (antigen binding fragment). The term "immunological reactivity" used herein indicates the characteristics of an antibody binding *in vivo* specifically to CAPRIN-1 protein or a partial polypeptide thereof.

The anti-CAPRIN-1 antibody used in the present invention may be a monoclonal antibody or a polyclonal antibody.

Polyclonal antibodies having an immunological reactivity with a full-length CAPRIN-1 protein or a fragment thereof (anti-CAPRIN-1 polyclonal antibodies) can be obtained, for example, in a manner described below. Serum is obtained from mice, human antibody-producing mice, rats, rabbits, chickens, or the like immunized with a naturally occurring CAPRIN-1 protein or a protein fused with GST or the like, or a partial peptide thereof, and the antibodies can be obtained by purifying the obtained serum via ammonium sulfate precipitation, protein A, protein G, DEAE ion-exchange columns, affinity columns to which CAPRIN-1 protein or a partial peptide is coupled, or the like.

As for nucleotide sequences and amino acid sequences of CAPRIN-1 and homologs thereof used in the immunization can be obtained by, for example, accessing the website of GenBank (NCBI, USA) and using the BLAST or FASTA algorithm (Karlin and Altschul, Proc. Natl. Acad. Sci. USA, 90:5873-5877, 1993; and Altschul et al., Nucleic Acids Res. 25:3389-3402, 1997). Methods for producing CAPRIN-1 protein can be obtained with reference to WO2014/012479 or may employ cells or the like expressing CAPRIN-1 protein.

Monoclonal antibodies having an immunological reactivity with a full-length CAPRIN-1 protein or a fragment thereof (anti-CAPRIN-1 monoclonal antibodies) can be obtained, for example, in a manner described below. Breast cancer cells SK-BR-3 expressing CAPRIN-1, a full-length CAPRIN-1 protein or a fragment thereof, or the like is administered to mice for immunization. Splenocytes separated from the mice are fused with myeloma cells. Clones capable of producing anti-CAPRIN-1 monoclonal antibodies can be selected from the obtained fusion cells (hybridomas) to obtain these antibodies. The antibodies produced from the selected hybridomas can be obtained in the same way as the aforementioned method for purifying polyclonal antibodies.

The antibody used in the present invention includes human antibodies, humanized antibodies, chimeric antibodies, and non-human animal antibodies.

For human antibodies, human lymphocytes infected with EB virus are sensitized with a protein, protein-expressing cells, or a lysate thereof. The sensitized lymphocytes are fused with human-derived myeloma cells such as U266 cells. Antibodies having an immunological reactivity with a full-length CAPRIN-1 protein or a fragment thereof can be obtained from the obtained fusion cells.

A humanized antibody is a modified antibody, and it is sometimes referred to as a reshaped human antibody. It is known that a humanized antibody is constructed by transplanting complementarity determining regions of an immunized animal-derived antibody into complementarity determining regions of a human antibody. In addition, a general gene recombinant technique therefor is well known. Specifically, a DNA sequence designed in a manner that allows complementarity determining regions of mouse or rabbit antibody to be ligated to human antibody framework regions is synthesized by the PCR method using several oligonucleotides prepared in such a manner that the oligonucleotides have portions overlapping each other at one end of each thereof. A humanized antibody can be obtained by ligating the above obtained DNA to DNA encoding a human antibody constant region, incorporating the resultant into an expression vector, and introducing the vector into a host for antibody production (see EP-A-239400 and WO96/02576). Framework regions of human antibody ligated to each other via complementarity determining regions are selected on the assumption that complementarity determining regions can form a good antigen binding site. If necessary, amino acids in framework regions of an antibody variable region may be substituted in such a manner that complementarity determining regions in a reshaped human antibody form an appropriate antigen binding site (Sato K. et al., Cancer Research 1993, 53:851-856). In addition, the framework regions may be substituted with framework regions from a different human antibody (see WO99/51743).

In general, antibodies are heteromultimeric glycoproteins each comprising at least two heavy chains and two light chains. Antibodies each comprise two identical light chains and two identical heavy chains. Each heavy chain has a heavy-chain variable region at one end thereof, to which some constant regions are bound in series. Each light chain has a light-chain variable region at one end thereof to which some constant regions are bound in series. Variable regions have a specific variable region, which is called complementarity determining region (CDR) and imparts binding specificity to an antibody. A relatively conserved portion in a variable region is called a framework region (FR). A complete heavy-chain or light-chain variable region comprises 4 FRs connected to each other via 3 CDRs (CDR1 to CDR3).

Sequences of human-derived heavy-chain and light-chain constant regions and variable regions can be obtained from, for example, NCBI (USA; GenBank, UniGene, etc.). For example, for a human IgG1 heavy-chain constant region, see registration No. J00228; for a human IgG2 heavy-chain constant region, see registration No. J00230; for a human light chain κ constant region, see sequences such as registration Nos. V00557, X64135, and X64133; and for a human light chain λ constant region, see sequences such as registration Nos. X64132 and X64134.

A chimeric antibody is an antibody produced by combining sequences from different animals. An example thereof is an antibody composed of mouse antibody heavy-chain and light-chain variable regions and human antibody heavy-chain and light-chain constant regions. Such a chimeric antibody can be produced by a known method. For example, it can be obtained by ligating DNA encoding an antibody V region to DNA encoding a human antibody C region, incorporating the resultant into an expression vector, and introducing the vector into a host for antibody production.

Non-human animal antibodies are obtained by immunizing animals with sensitizing antigens according to a known method or by intraperitoneally, intracutaneously, or subcutaneously injecting sensitizing antigens into animals such as mice as a general method. For injecting sensitizing antigens, an appropriate amount of various adjuvants including CFA (Freund's complete adjuvant) is mixed therewith and the mixture is administered to animals several times. After immunization of animals and confirmation of an anti-CAPRIN-1 antibody contained in serum, the serum is obtained and the non-human animal antibody can be obtained by purification via ammonium sulfate precipitation, protein A, protein G, DEAE ion-exchange columns, affinity columns to which CAPRIN-1 protein or a partial peptide is coupled, or the like, as mentioned above. In the case of obtaining monoclonal antibodies from non-human animals, a monoclonal antibody is obtained by collecting immunocytes from the immunized animals and subjected to cell fusion with myeloma cells. The cell fusion of immunocytes with myeloma cells can be carried out according to a known method (see Kohler, G. and Milstein, C. Methods Enzymol. (1981) 73, 3-46).

The antibody used in the present invention can also be obtained as a gene recombinant antibody produced by cloning an antibody gene from a hybridoma, incorporating the clone into an adequate vector, introducing the vector into a host, and producing the antibody by using a gene recombinant technique (see Carl, A.K. Borrebaeck, James, W. Larrick, THERAPEUTIC MONOCLONAL ANTIBODIES, Published in the United Kingdom by MACMILLAN PUBLISHERS LTD, 1990).

Amino acids in a variable region (e.g., FR) or a constant region in the anti-CAPRIN-1 antibody used in the present invention may be substituted with different amino acids. The amino acid substitution is a substitution of 1 or several, for example, less than 15, less than 10, not more than 8, not more than 6, not more than 5, not more than 4, not more than 3, or not more than 2 amino acids, preferably 1 to 9 amino acids. A substituted antibody should have characteristics of specifically binding to the antigen and binding affinity for the antigen equivalent to or more than those of an unsubstituted antibody and should be an antibody that causes no rejection when applied to humans. The amino acid substitution is preferably a conservative amino acid substitution, which is a substitution between amino acids having similar characteristics in terms of charge, side chains, polarity, aromaticity, and the like. For example, characteristically similar amino acids can be classified into the following types: basic amino acids (arginine, lysine, and histidine); acidic amino acids (aspartic acid and glutamic acid); uncharged polar amino acids (glycine, asparagine, glutamine, serine, threonine, cysteine, and tyrosine); nonpolar amino acids (leucine, isoleucine, alanine, valine, proline, phenylalanine, tryptophan, and methionine); branched-chain amino acids (threonine, valine, isoleucine); and aromatic amino acids (phenylalanine, tyrosine, tryptophan, and histidine).

The anti-CAPRIN-1 antibody used in the present invention is expected to have a stronger antitumor effect when having higher binding affinity for CAPRIN-1 protein on cancer cell surfaces. Association constant (affinity constant) Ka (kon/koff) is preferably at least 10⁷ M⁻¹, at least 10⁸ M⁻¹, at least 5 × 10⁸ M⁻¹, at least 10⁹ M⁻¹, at least 5 × 10⁹ M⁻¹, at least 10¹⁰ M⁻¹, at least 5 × 10¹⁰ M⁻¹, at least 10¹¹ M⁻¹, at least 5 × 10¹¹ M⁻¹, at least 10¹² M⁻¹, or at least 10¹³ M⁻¹.

The ability of an anti-CAPRIN-1 antibody to bind to CAPRIN-1 can be determined via binding assay using, for example, ELISA, a Western blot method, immunofluorescence, or flowcytometry analysis.

The anti-CAPRIN-1 antibody used in the present invention may be chemically modified. Examples of such an antibody modifier can include antibodies bound to various molecules such as polyethylene glycol (PEG) and antitumor compounds (for example, antitumor agents listed below). Regarding antibody modifiers of the present invention, substances that bind to an antibody are not limited. Such an antibody modifier can be obtained by chemically modifying an obtained antibody. Methods of such modification have been already established in the field related to the present invention.

The binding strength of the anti-CAPRIN-1 antibody used in the present invention against effector cells can be improved by substituting 1, 2 or several amino acids in the heavy-chain constant region of the antibody or by removing fucose bound to N-acetylglucosamine in an N-glycoside-linked sugar chain bound to the heavy-chain constant region. The anti-CAPRIN-1 antibody described above may have the amino acid substitution alone or may be a composition with an antibody bound to fucose.

Antibodies in which 1, 2 or several amino acids in the heavy-chain constant region have been substituted can be produced with reference to, for example, WO2004/063351, WO2011/120135, U.S. Patent No. 8388955, WO2011/005481, U.S. Patent No. 6737056, and WO2005/063351.

Antibodies in which fucose bound to N-acetylglucosamine in an N-glycoside-linked sugar chain in the heavy-chain constant region has been removed, or producing cells thereof can be produced with reference to U.S. Patent No. 6602684, EP Patent No. 1914244, and U.S. Patent No. 7579170. Compositions or producing cells of antibodies in which fucose bound to *N-*acetylglucosamine in an N-glycoside-linked sugar chain bound to the heavy-chain constant region has been removed, with antibodies bound to fucose can be produced with reference to, for example, U.S. Patent No. 8642292.

The anti-CAPRIN-1 polyclonal antibody and the anti-CAPRIN-1 monoclonal antibody used in the present invention, methods for producing or purifying antibodies and methods for producing CAPRIN-1 protein or partial polypeptide thereof used in immunization can be obtained with reference to WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212.

Specific examples of the anti-CAPRIN-1 antibody according to the present invention include anti-CAPRIN-1 antibodies described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212 mentioned above. Preferred examples of the anti-CAPRIN-1 antibody include the following.

An antibody or a fragment thereof having an immunological reactivity with a partial polypeptide of CAPRIN-1 protein having the amino acid sequence shown by SEQ ID NO: 2 or SEQ ID NO: 4 or an amino acid sequence having 80% or more (preferably 85% or more, more preferably 90% or more, further preferably 95% or more, and still further preferably 99% or more) sequence identity to the amino acid sequence.

An antibody or a fragment thereof having an immunological reactivity with a partial polypeptide of CAPRIN-1 protein, the partial polypeptide having the amino acid sequence shown by SEQ ID NO: 31 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 36, 37 and 38 (CDR1, CDR2 and CDR3, respectively) and a light-chain variable region comprising complementarity determining regions of SEQ ID NOs: 40, 41 and 42 (CDR1, CDR2 and CDR3, respectively), and having an immunological reactivity with CAPRIN-1 protein; an antibody or a fragment thereof comprising a heavy-chain variable region comprising complementarity determining regions of SEQ ID NOs: 140, 141 and 142 (CDR1, CDR2 and CDR3, respectively) and a light-chain variable region comprising complementarity determining regions of SEQ ID NOs: 143, 144 and 145 (CDR1, CDR2 and CDR3, respectively), and having an immunological reactivity with CAPRIN-1 protein; or an antibody or a fragment thereof comprising a heavy-chain variable region comprising complementarity determining regions of SEQ ID NOs: 164, 165 and 166 (CDR1, CDR2 and CDR3, respectively) and a light-chain variable region comprising complementarity determining regions of SEQ ID NOs: 167, 168 and 169 (CDR1, CDR2 and CDR3, respectively), and having an immunological reactivity with CAPRIN-1 protein. More preferably, an antibody or a fragment thereof comprising a heavy-chain variable region comprising the amino acid sequence of SEQ ID NO: 39 and a light-chain variable region comprising the amino acid sequence of SEQ ID NO: 43; an antibody or a fragment thereof comprising a heavy-chain variable region comprising the amino acid sequence of SEQ ID NO: 70 and a light-chain variable region comprising the amino acid sequence of SEQ ID NO: 71; or an antibody or a fragment thereof comprising a heavy-chain variable region comprising the amino acid sequence of SEQ ID NO: 78 and a light-chain variable region comprising the amino acid sequence of SEQ ID NO: 79.

An antibody or a fragment thereof having an immunological reactivity with a partial polypeptide of CAPRIN-1 protein, the partial polypeptide having the amino acid sequence shown by SEQ ID NO: 33 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 60, 61 and 62 (CDR1, CDR2 and CDR3, respectively) and a light-chain variable region comprising complementarity determining regions of SEQ ID NOs: 64, 65 and 66 (CDR1, CDR2 and CDR3, respectively), and having an immunological reactivity with CAPRIN-1 protein. More preferably, an antibody or a fragment thereof comprising a heavy-chain variable region comprising the amino acid sequence of SEQ ID NO: 63 and a light-chain variable region comprising the amino acid sequence of SEQ ID NO: 67.

An antibody or a fragment thereof having an immunological reactivity with a partial polypeptide of CAPRIN-1 protein, the partial polypeptide having the amino acid sequence shown by SEQ ID NO: 32 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 52, 53 and 54 (CDR1, CDR2 and CDR3, respectively) and a light-chain variable region comprising complementarity determining regions of SEQ ID NOs: 56, 57 and 58 (CDR1, CDR2 and CDR3, respectively), and having an immunological reactivity with CAPRIN-1 protein. More preferably, an antibody or a fragment thereof comprising a heavy-chain variable region comprising the amino acid sequence of SEQ ID NO: 55 and a light-chain variable region comprising the amino acid sequence of SEQ ID NO: 59.

An antibody or a fragment thereof having an immunological reactivity with a partial polypeptide of CAPRIN-1 protein, the partial polypeptide having the amino acid sequence shown by SEQ ID NO: 34 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 170, 171 and 172 (CDR1, CDR2 and CDR3, respectively) and a light-chain variable region comprising complementarity determining regions of SEQ ID NOs: 173, 174 and 175 (CDR1, CDR2 and CDR3, respectively), and having an immunological reactivity with CAPRIN-1 protein; or an antibody or a fragment thereof comprising a heavy-chain variable region comprising complementarity determining regions of SEQ ID NOs: 176, 177 and 178 (CDR1, CDR2 and CDR3, respectively) and a light-chain variable region comprising complementarity determining regions of SEQ ID NOs: 179, 180 and 181 (CDR1, CDR2 and CDR3, respectively), and having an immunological reactivity with CAPRIN-1 protein. More preferably, an antibody or a fragment thereof comprising a heavy-chain variable region comprising the amino acid sequence of SEQ ID NO: 80 and a light-chain variable region comprising the amino acid sequence of SEQ ID NO: 81; or an antibody or a fragment thereof comprising a heavy-chain variable region comprising the amino acid sequence of SEQ ID NO: 82 and a light-chain variable region comprising the amino acid sequence of SEQ ID NO: 83.

An antibody or a fragment thereof having an immunological reactivity with a partial polypeptide of CAPRIN-1 protein, the partial polypeptide having the amino acid sequence shown by SEQ ID NO: 35 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 182, 183 and 184 (CDR1, CDR2 and CDR3, respectively) and a light-chain variable region comprising complementarity determining regions of SEQ ID NOs: 185, 186 and 187 (CDR1, CDR2 and CDR3, respectively), and having an immunological reactivity with CAPRIN-1 protein; or an antibody or a fragment thereof comprising a heavy-chain variable region comprising complementarity determining regions of SEQ ID NOs: 188, 189 and 190 (CDR1, CDR2 and CDR3, respectively) and a light-chain variable region comprising complementarity determining regions of SEQ ID NOs: 191, 192 and 193 (CDR1, CDR2 and CDR3, respectively), and having an immunological reactivity with CAPRIN-1 protein. More preferably, an antibody or a fragment thereof comprising a heavy-chain variable region comprising the amino acid sequence of SEQ ID NO: 84 and a light-chain variable region comprising the amino acid sequence of SEQ ID NO: 85; or an antibody or a fragment thereof comprising a heavy-chain variable region comprising the amino acid sequence of SEQ ID NO: 86 and a light-chain variable region comprising the amino acid sequence of SEQ ID NO: 87.

An antibody or a fragment thereof comprising a heavy-chain variable region comprising complementarity determining regions of SEQ ID NOs: 44, 45 and 46 (CDR1, CDR2 and CDR3, respectively) and a light-chain variable region comprising complementarity determining regions of SEQ ID NOs: 48, 49 and 50 (CDR1, CDR2 and CDR3, respectively), and having an immunological reactivity with CAPRIN-1 protein. Preferably, an antibody or a fragment thereof comprising a heavy-chain variable region comprising the amino acid sequence of SEQ ID NO: 47 and a light-chain variable region comprising the amino acid sequence of SEQ ID NO: 51.

An antibody or a fragment thereof having an immunological reactivity with a partial polypeptide of CAPRIN-1 protein, the partial polypeptide having the amino acid sequence shown by SEQ ID NO: 296 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 146, 147 and 148 (CDR1, CDR2 and CDR3, respectively) and a light-chain variable region comprising complementarity determining regions of SEQ ID NOs: 149, 150 and 151 (CDR1, CDR2 and CDR3, respectively), and having an immunological reactivity with CAPRIN-1 protein. More preferably, an antibody or a fragment thereof comprising a heavy-chain variable region comprising the amino acid sequence of SEQ ID NO: 72 and a light-chain variable region comprising the amino acid sequence of SEQ ID NO: 73.

An antibody or a fragment thereof having an immunological reactivity with a partial polypeptide of CAPRIN-1 protein, the partial polypeptide having the amino acid sequence shown by SEQ ID NO: 297 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 272, 273 and 274 (CDR1, CDR2 and CDR3, respectively) and a light-chain variable region comprising complementarity determining regions of SEQ ID NOs: 275, 276 and 277 (CDR1, CDR2 and CDR3, respectively), and having an immunological reactivity with CAPRIN-1 protein. More preferably, an antibody or a fragment thereof comprising a heavy-chain variable region comprising the amino acid sequence of SEQ ID NO: 114 and a light-chain variable region comprising the amino acid sequence of SEQ ID NO: 115.

An antibody or a fragment thereof having an immunological reactivity with a partial polypeptide of CAPRIN-1 protein, the partial polypeptide having the amino acid sequence shown by SEQ ID NO: 298 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 290, 291 and 292 (CDR1, CDR2 and CDR3, respectively) and a light-chain variable region comprising complementarity determining regions of SEQ ID NOs: 293, 294 and 295 (CDR1, CDR2 and CDR3, respectively), and having an immunological reactivity with CAPRIN-1 protein. More preferably, an antibody or a fragment thereof comprising a heavy-chain variable region comprising the amino acid sequence of SEQ ID NO: 120 and a light-chain variable region comprising the amino acid sequence of SEQ ID NO: 121.

An antibody or a fragment thereof having an immunological reactivity with a partial polypeptide of CAPRIN-1 protein, the partial polypeptide having the amino acid sequence shown by SEQ ID NO: 299 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 300, 301 and 302 (CDR1, CDR2 and CDR3, respectively) and a light-chain variable region comprising complementarity determining regions of SEQ ID NOs: 304, 305 and 306 (CDR1, CDR2 and CDR3, respectively), and having an immunological reactivity with CAPRIN-1 protein. More preferably, an antibody or a fragment thereof comprising a heavy-chain variable region comprising the amino acid sequence of SEQ ID NO: 300 and a light-chain variable region comprising the amino acid sequence of SEQ ID NO: 304.

An antibody or a fragment thereof having an immunological reactivity with a partial polypeptide of CAPRIN-1 protein, the partial polypeptide having the amino acid sequence shown by SEQ ID NO: 308 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein. More preferably, an antibody or a fragment thereof comprising a heavy-chain variable region comprising the amino acid sequence of SEQ ID NO: 68 and a light-chain variable region comprising the amino acid sequence of SEQ ID NO: 69.

An antibody or a fragment thereof having an immunological reactivity with a partial polypeptide of CAPRIN-1 protein, the partial polypeptide having the amino acid sequence shown by SEQ ID NO: 309 or an amino acid sequence having 80% or more (preferably 85% or more, more preferably 90% or more, and further preferably 95% or more) sequence identity to the amino acid sequence. Preferably, an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein. More preferably, an antibody or a fragment thereof comprising a heavy-chain variable region comprising the amino acid sequence of SEQ ID NO: 68 and a light-chain variable region comprising the amino acid sequence of SEQ ID NO: 69.

In addition, the following anti-CAPRIN-1 antibodies are preferably used.

An antibody or a fragment thereof comprising a heavy-chain variable region comprising the amino acid sequence of SEQ ID NO: 68 and a light-chain variable region comprising the amino acid sequence of SEQ ID NO: 69.

An antibody or a fragment thereof comprising a heavy-chain variable region comprising the amino acid sequence of SEQ ID NO: 70 and a light-chain variable region comprising the amino acid sequence of SEQ ID NO: 71.

An antibody or a fragment thereof comprising a heavy-chain variable region comprising the amino acid sequence of SEQ ID NO: 72 and a light-chain variable region comprising the amino acid sequence of SEQ ID NO: 73.

An antibody or a fragment thereof comprising a heavy-chain variable region comprising the amino acid sequence of SEQ ID NO: 74 and a light-chain variable region comprising the amino acid sequence of SEQ ID NO: 75.

An antibody or a fragment thereof comprising a heavy-chain variable region comprising the amino acid sequence of SEQ ID NO: 76 and a light-chain variable region comprising the amino acid sequence of SEQ ID NO: 77.

An antibody or a fragment thereof comprising a heavy-chain variable region comprising the amino acid sequence of SEQ ID NO: 78 and a light-chain variable region comprising the amino acid sequence of SEQ ID NO: 79.

An antibody or a fragment thereof comprising a heavy-chain variable region comprising the amino acid sequence of SEQ ID NO: 80 and a light-chain variable region comprising the amino acid sequence of SEQ ID NO: 81.

An antibody or a fragment thereof comprising a heavy-chain variable region comprising the amino acid sequence of SEQ ID NO: 82 and a light-chain variable region comprising the amino acid sequence of SEQ ID NO: 83.

An antibody or a fragment thereof comprising a heavy-chain variable region comprising the amino acid sequence of SEQ ID NO: 84 and a light-chain variable region comprising the amino acid sequence of SEQ ID NO: 85.

An antibody or a fragment thereof comprising a heavy-chain variable region comprising the amino acid sequence of SEQ ID NO: 86 and a light-chain variable region comprising the amino acid sequence of SEQ ID NO: 87.

An antibody or a fragment thereof comprising a heavy-chain variable region comprising the amino acid sequence of SEQ ID NO: 88 and a light-chain variable region comprising the amino acid sequence of SEQ ID NO: 89.

An antibody or a fragment thereof comprising a heavy-chain variable region comprising the amino acid sequence of SEQ ID NO: 90 and a light-chain variable region comprising the amino acid sequence of SEQ ID NO: 91.

An antibody or a fragment thereof comprising a heavy-chain variable region comprising the amino acid sequence of SEQ ID NO: 92 and a light-chain variable region comprising the amino acid sequence of SEQ ID NO: 93.

An antibody or a fragment thereof comprising a heavy-chain variable region comprising the amino acid sequence of SEQ ID NO: 94 and a light-chain variable region comprising the amino acid sequence of SEQ ID NO: 95.

An antibody or a fragment thereof comprising a heavy-chain variable region comprising the amino acid sequence of SEQ ID NO: 96 and a light-chain variable region comprising the amino acid sequence of SEQ ID NO: 97.

An antibody or a fragment thereof comprising a heavy-chain variable region comprising the amino acid sequence of SEQ ID NO: 98 and a light-chain variable region comprising the amino acid sequence of SEQ ID NO: 99.

An antibody or a fragment thereof comprising a heavy-chain variable region comprising the amino acid sequence of SEQ ID NO: 100 and a light-chain variable region comprising the amino acid sequence of SEQ ID NO: 101.

An antibody or a fragment thereof comprising a heavy-chain variable region comprising the amino acid sequence of SEQ ID NO: 102 and a light-chain variable region comprising the amino acid sequence of SEQ ID NO: 103.

An antibody or a fragment thereof comprising a heavy-chain variable region comprising the amino acid sequence of SEQ ID NO: 104 and a light-chain variable region comprising the amino acid sequence of SEQ ID NO: 105.

An antibody or a fragment thereof comprising a heavy-chain variable region comprising the amino acid sequence of SEQ ID NO: 106 and a light-chain variable region comprising the amino acid sequence of SEQ ID NO: 107.

An antibody or a fragment thereof comprising a heavy-chain variable region comprising the amino acid sequence of SEQ ID NO: 108 and a light-chain variable region comprising the amino acid sequence of SEQ ID NO: 109.

An antibody or a fragment thereof comprising a heavy-chain variable region comprising the amino acid sequence of SEQ ID NO: 110 and a light-chain variable region comprising the amino acid sequence of SEQ ID NO: 111.

An antibody or a fragment thereof comprising a heavy-chain variable region comprising the amino acid sequence of SEQ ID NO: 112 and a light-chain variable region comprising the amino acid sequence of SEQ ID NO: 113.

An antibody or a fragment thereof comprising a heavy-chain variable region comprising the amino acid sequence of SEQ ID NO: 114 and a light-chain variable region comprising the amino acid sequence of SEQ ID NO: 115.

An antibody or a fragment thereof comprising a heavy-chain variable region comprising the amino acid sequence of SEQ ID NO: 116 and a light-chain variable region comprising the amino acid sequence of SEQ ID NO: 117.

An antibody or a fragment thereof comprising a heavy-chain variable region comprising the amino acid sequence of SEQ ID NO: 118 and a light-chain variable region comprising the amino acid sequence of SEQ ID NO: 119.

An antibody or a fragment thereof comprising a heavy-chain variable region comprising the amino acid sequence of SEQ ID NO: 120 and a light-chain variable region comprising the amino acid sequence of SEQ ID NO: 121.

An antibody or a fragment thereof comprising a heavy-chain variable region comprising the amino acid sequence of SEQ ID NO: 122 and a light-chain variable region comprising the amino acid sequence of SEQ ID NO: 123.

An antibody or a fragment thereof comprising a heavy-chain variable region comprising the amino acid sequence of SEQ ID NO: 124 and a light-chain variable region comprising the amino acid sequence of SEQ ID NO: 125.

An antibody or a fragment thereof comprising a heavy-chain variable region comprising the amino acid sequence of SEQ ID NO: 126 and a light-chain variable region comprising the amino acid sequence of SEQ ID NO: 127.

An antibody or a fragment thereof comprising a heavy-chain variable region comprising the amino acid sequence of SEQ ID NO: 128 and a light-chain variable region comprising the amino acid sequence of SEQ ID NO: 129.

An antibody or a fragment thereof comprising a heavy-chain variable region comprising the amino acid sequence of SEQ ID NO: 130 and a light-chain variable region comprising the amino acid sequence of SEQ ID NO: 131.

An antibody or a fragment thereof comprising a heavy-chain variable region comprising the amino acid sequence of SEQ ID NO: 132 and a light-chain variable region comprising the amino acid sequence of SEQ ID NO: 133.

An antibody or a fragment thereof comprising a heavy-chain variable region comprising the amino acid sequence of SEQ ID NO: 303 and a light-chain variable region comprising the amino acid sequence of SEQ ID NO: 307.

In Examples mentioned later, a polyclonal antibody or a monoclonal antibody against full-length CAPRIN-1 protein or a polypeptide of a portion of a region expressed on cell membrane surfaces of cancer cells, combined with a drug comprising an anthracycline-based drug and a platinum formulation, or an anthracycline-based drug, a platinum formulation, and an angiogenesis inhibitor, was confirmed to have its strong antitumor effect in cancer-bearing organisms.

### <Anthracycline-based Drug>

Although not particularly limited, the anthracycline-based drug includes doxorubicin, liposomal doxorubicin, aclarubicin, amrubicin, epirubicin, zinostatin stimalamer, daunorubicin, pirarubicin, bleomycin, peplomycin, mitomycin C, mitoxantrone, actinomycin D, an idarubicin formulation, and pharmacologically acceptable salts thereof or derivatives thereof, preferably doxorubicin or a doxorubicin hydrochloride liposome formulation (PLD), more preferably PLD.

### <Platinum Formulation>

The platinum formulation is a metal complex in which the central metal is composed of platinum, four ligands are on the same plane, and an amine as one of the ligands forms a cis form. The platinum formulation is a drug that exerts an antitumor effect when coordinated to a guanine base of a DNA, and may comprise a suitable isotonizing agent or a pH adjusting agent or a suitable dosage form to be administered to an organism. Specific examples of the platinum formulation include, but are not particularly limited to, carboplatin, cisplatin, oxaliplatin, nedaplatin, and miriplatin, and preferably carboplatin.

### <Angiogenesis inhibitor>

The angiogenesis inhibitor is a drug that inhibits the migration and growth of a vascular endothelial cell, and inhibits angiogenesis, and is preferably an anti-VEGF inhibitor and/or an anti-vascular endothelial growth factor receptor (VEGFR) inhibitor. Specific examples of the anti-VEGF inhibitor include, but are not particularly limited to, Bevacizumab, Ranibizumab, and Ziv-Aflibercept, and specific examples of the anti-vascular endothelial growth factor receptor (VEGFR) inhibitor include Ramucirumab, Axitinib, Cabozantinib, Regorafenib, Sorafenib, Sunitinib, Vandetanib, Pazopanib and Lenvatinib mesilate Bevacizumab is preferable.

### <Another Drug>

A medicament according to the present invention may further be used in combination with an antitumor agent known in literature, etc. However, it is characterized in that a pyrimidine-based drug is not used in combination from the viewpoint of safety. The pyrimidine-based drug includes fluorouracil (5-FU) or prodrugs of fluorouracil such as tegafur, tegafur uracil (UFT), tegafur/gimeracil/oteracil potassium (S-1), capecitabine, carmofur, floxuridine, gemcitabine (GEM), doxifluridine, and a cytarabine formulation, or derivatives thereof.

Specific examples of known antitumor agents that can be used in combination with a medicament according to the present invention include, but are not particularly limited to, paclitaxel, nab-paclitaxel, cyclophosphamide, methotrexate, thiotepa, busulfan, improsulfan, piposulfan, benzodopa, carboquone, meturedopa, uredopa, altretamine, triethylenemelamine, triethylenephosphoramide, triethylenethiophosphoramide, trimethylolomelamine, bullatacin, bullatacinone, camptothecin, bryostatin, callystatin, cryptophycin 1, cryptophycin 8, dolastatin, duocarmycin, eleutherobin, pancratistatin, sarcodictyin, spongistatin, chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard, carmustine, chlorozotocin, fotemustine, lomustine, nimustine, ranimustine, calicheamicin, dynemicin, clodronate, esperamicin, aclacinomycin, authramycin, azaserine, cactinomycin, carabicin, carminomycin, carzinophilin, chromomycin, dactinomycin, detorbicin, 6-diazo-5-oxo-L-norleucine, esorubicin, marcellomycin, mycophenolic acid, nogalamycin, olivomycin, potfiromycin, puromycin, quelamycin, rodorubicin, streptonigrin, streptozocin, tubercidin, ubenimex, zinostatin, zorubicin, denopterin, pteropterin, trimetrexate, thiamiprine, thioguanine, ancitabine, 6-azauridine, dideoxyuridine, floxuridine; androgens, for example, calusterone, dromostanolone propionate, epitiostanol, mepitiostane, testolactone, aminoglutethimide, mitotane, trilostane, frolinic acid, aceglatone, aldophosphamide glycoside, aminolevulinic acid, eniluracil, amsacrine, bestrabucil, bisantrene, defofamine, demecolcine, diaziquone, eflornithine, elliptinium acetate, epothilone, etoglucid, lentinan, lonidamine, maytansine, ansamitocine, mitoguazone, mopidanmol, nitraerine, phenamet, losoxantrone, podophyllinic acid, 2-ethylhydrazide, procarbazine, razoxane, rhizoxin, schizophyllan, spirogermanium, tenuazonic acid, triaziquone, roridine A, anguidine, urethane, vindesine, dacarbazine, mannomustine, mitobronitol, pipobroman, gacytosine, docetaxel, 6-thioguanine, mercaptopurine, vinblastine, etoposide, mitoxantrone, vincristine, vinorelbine, novantrone, teniposide, edatrexate, daunomycin, aminopterin, ibandronate, irinotecan, topoisomerase inhibitor, difluoromethylornithine (DMFO), retinoic acid, and pharmacologically acceptable (known) salts or (known) derivatives thereof.

### <Antitumor effect of present invention>

The antitumor effect can be evaluated by examining the antitumor effect of a medicament according to the present invention on cancer *in vivo* or *in vitro.* As a specific example, the antitumor effect can be evaluated by administering the medicament according to the present invention to an organism having cancer, measuring the size of a tumor after the administration, and examining the size of the cancer over time. Also, the antitumor effect can be evaluated by examining the survival rate of an organism having cancer after the administration of the medicament according to the present invention. Alternatively, the antitumor effect of the present invention may be evaluated by examining the ability to produce cytokines or chemokines. The antitumor effect can be further evaluated by examining the prevention of cancer, prevention of metastasis or prevention of recurrence.

Administering, to an organism having cancer, a medicament according to the present invention, increases an antitumor effect as compared with an anti-CAPRIN-1 antibody alone, as mentioned above. The rate of increase is preferably 30% or more, more preferably 40% or more, further preferably 50% or more, still further preferably 55% or more, even further preferably 60% or more, even further preferably 65% or more, and most preferably 70% or more. The rate of increase in antitumor effect by administration of a medicament according to the present invention, with respect to administration of the anti-CAPRIN-1 antibody alone can be calculated by administering their respective effective amounts to cancer-bearing mice under the same conditions, and comparing tumor volumes on 7 days or later after the start of administration.

### <Medicament for treatment and/or prevention of cancer>

A medicament of the present invention is aimed at treating and/or preventing cancer. A cancer targeted by the medicament of the present invention is not particularly limited as long as it is a cancer (cells) expressing CAPRIN-1 protein.

The term "treatment" used herein refers to treatment of cancer based on an antitumor effect mentioned above. The term "prevention" used herein refers to not only prevention of development of cancer but prevention of metastasis or recurrence of cancer.

Both the terms "tumor" and "cancer" used herein refer to malignant neoplasm, and thus they are used in an exchangeable manner.

Cancer that can be a target in the present invention is any cancer as long as the cancer expresses CAPRIN-1 protein on a cell membrane surface. The cancer is preferably ovarian cancer, bile duct cancer, breast cancer, kidney cancer, pancreatic cancer, colon cancer, melanoma (including postoperative melanoma), lung cancer (including non-small-cell lung cancer and small cell lung cancer), renal cell carcinoma, Hodgkin's lymphoma, head and neck cancer, gastric cancer, mesothelioma (including malignant pleural mesothelioma), colorectal cancer (for example, colorectal cancer having MSI-high), esophageal cancer, gastroesophageal junction cancer, hepatocellular carcinoma, glioblastoma, urothelial carcinoma, urinary bladder cancer, uterus cancer (including uterine cervical cancer and uterine body cancer), primary central nervous system lymphoma, primary testicular lymphoma, biliary tract cancer, brain tumor, prostate cancer, leukemia, lymphoma, liver cancer, sarcoma, fibrosarcoma, mastocytoma, adrenocortical carcinoma, Ewing's tumor, multiple myeloma, testicular cancer, thyroid cancer, basal cell carcinoma, Paget's disease, or skin cancer. In addition, these cancers may be primary cancers, metastatic cancers, cancers that have metastasized, cancers that have recurred, postoperative cancers, or unresectable cancers. In this regard, melanoma is often used synonymously with malignant melanoma.

Other examples of a cancer targeted in the present invention include a cancer resistant to a known treatment method. The cancer having resistance is not particularly limited and may be a cancer derived from a patient having any history of treatment. The cancer is, for example, a cancer derived from a patient having a history of treatment with 5-FU, which has come to have resistance, the cancer metastasized, or the cancer recurred after the administration.

More specifically, examples of the cancer include, but are not limited to, for example, ovarian epithelial cancer, germ cell tumor, sex cord-stromal tumor, primary peritoneal carcinoma, Bowen's disease, melanoma, prickle cell cancer, extramammary Paget's disease, mycosis fungoides, Sezary's syndrome, cutaneous T/NK-cell lymphoma, T-cell leukemia or lymphoma having a lesion only in the skin, cutaneous B-cell lymphoma (indolent group), cutaneous T-cell lymphatic or mammary adenoma, complex mammary adenoma, malignant mixed tumor of mammary gland, intraductal papillary carcinoma of mammary gland, lung adenocarcinoma, squamous cell cancer, small cell cancer, large cell cancer, glioma which is a neuroepithelial tissue tumor, glioblastoma, neuroblastoma, ependymoma, neuronal tumor, embryonal neuroectodermal tumor, neurilemoma, neurofibromatosis, meningioma, chronic lymphocytic leukemia, lymphoma, alimentary lymphoma, gastrointestinal lymphoma, small to medium cell lymphoma, cecal cancer, ascending colon cancer, descending colon cancer, transverse colon cancer, sigmoid colon cancer, rectal cancer, interstitial cell tumor, pancreatic duct cancer, invasive pancreatic duct cancer, adenocarcinoma of pancreatic cancer, acinar cell cancer, adenosquamous cancer, giant cell tumor, intraductal papillary mucinous tumor, mucinous cystadenocarcinoma, pancreatoblastoma, pancreatic islet cell tumor, Frantz's tumor, serous cystadenocarcinoma, solid pseudopapillary cancer, gastrinoma, glucagonoma, insulinoma, multiple endocrine neoplasia type-1 (Wermer syndrome), nonfunctional islet cell tumor, somatostatinoma, VIPoma, uterine cervical cancer, uterine body cancer, fibrosarcoma, osteosarcoma, joint sarcoma, Ewing sarcoma, Wilms's tumor, hepatoblastoma, soft tissue sarcoma, acute leukemia, chronic leukemia, spinal cord tumor, malignant soft tissue tumor, tumors of teratoma group, and head and neck cancer including hypopharynx cancer, oropharynx cancer, tongue cancer, nasopharyngeal cancer, oral cavity cancer, lip cancer, sinus cancer, voice box cancer, ureteropelvic cancer, urinary bladder cancer, urethral cancer, testicular neoplasm, malignant pleural mesothelioma, malignant bone tumor, uterine body cancer, pediatric malignant solid tumor (rhabdomyosarcoma, neuroblastoma, hepatoblastoma, medulloblastoma, nephroblastoma, retinoblastoma, central nervous system germ cell tumor, and Ewing sarcoma family of tumors), serous tumor (encompassing serous borderline malignant tumor (SBT)), mucinous tumor, surface epithelial borderline malignant tumor, interstitial borderline malignant tumor, and the like. The cancer also includes a palpable cancer, a subcutaneously existing cancer, an intracutaneously existing cancer, a superficial cancer, cancer existing in the dermis, cancer existing in a non-parenchymal organ, and a progressive cancer, which originate from the cancers described above. The cancer also includes a palpable cancer, a subcutaneously existing cancer, an intracutaneously existing cancer, a superficial cancer, cancer existing in the dermis and cancer existing in a non-parenchymal organ, which originate from the cancers described above and have metastasized and recurred.

Although not particularly limited, cancer patients to whom the medicament according to the present invention is administered include, as a specific example, a cancer patient having a medical history of cancer treatment with a medicament other than a cancer treatment with the medicament according to the present invention. The cancer patient also encompasses a cancer patient who was treated with a chemotherapeutic, a molecular target drug, or a hormone therapy in the past, and a cancer patient who had cancer treatment in accordance with "NCCN Clinical Practice Guidelines in Oncology", "ESMO Clinical Practice Guidelines", or "Clinical Practice Guideline on Cancer". The cancer patient preferably has a medical history of cancer treatment with at least one of an anthracycline-based drug (for example, doxorubicin or a doxorubicin hydrochloride liposome formulation (PLD)), a platinum formulation (for example, carboplatin, cisplatin, oxaliplatin, nedaplatin, or miriplatin), and an angiogenesis inhibitor (for example, bevacizumab), and more preferably has sensitivity to a platinum formulation.

In addition, the cancer patient having a medical history is preferably a cancer patient having a cancer with a resistance to a cancer treatment with a medicament other than a cancer treatment with a medicament according to the present invention.

A preferable subject (patient) that can be a target is a mammal and is, for example, a mammal including primates, pet animals, livestock animals, and sport animals. Humans, dogs and cats are particularly preferable.

A medicament of the present invention can be formulated by a method known to persons skilled in the art. For instance, a medicament according to the present invention can be parenterally used in the form of a parenteral injection of: an aseptic solution in water or a pharmacologically acceptable non-water solution; or a suspension liquid. For each formulation or pharmaceutical composition in a medicament according to the present invention, the active ingredients thereof (at least one of an anti-CAPRIN-1 antibody, an anthracycline-based drug, a platinum formulation, or an angiogenesis inhibitor) may be combined, for example, with a pharmacologically acceptable carrier, a medium, or an additive, specifically sterilized water, physiological saline, an isotonic solution, a buffering agent (buffer solution or the like), plant oil, oily solution, an antioxidant, a dissolution aid, an emulsifier, a suspension, a surfactant, a stabilizer, a fragrance, an excipient, a binder, or the like in an appropriate manner, and preferably may be mixed with such a component in a unit dosage form required for a generally acceptable pharmaceutical formulation. An amount of an active ingredient in a formulation is determined such that an appropriate dosage within an indicated range can be achieved.

An aseptic composition for injection can be prepared in accordance with general formulation practice using a vehicle such as distilled water for injection. An aqueous solution for injection purposes includes, for example, physiological saline or isotonic solutions comprising glucose and other adjuvants such as D-sorbitol, D-mannose, D-mannitol, and sodium chloride. Such solution may be used with an appropriate dissolution aid. Such dissolution aid includes, for example, alcohols such as ethanol and polyalcohol, such as propylene glycol, polyethylene glycol, or nonion surfactants such as polysorbate 80(TM) and HCO-60. Oily liquid includes, for example, sesame oil or soybean oil. Such oily liquid may be used in combination with a dissolution aid such as benzyl benzoate or benzyl alcohol. In addition, it may be mixed with a buffering agent such as a phosphate buffer solution or a sodium acetate buffer solution, a soothing agent such as procaine hydrochloride, a stabilizer such as benzyl alcohol or phenol, or an antioxidant. In general, a formulated injection solution is introduced into an adequate ampule.

The above pharmaceutical composition is orally or parenterally administered. Preferably, it is parenterally administered. Specifically, dosage forms include forms of injectable agents, intranasally-administered agents, transpulmonarily-administered agents, and percutaneously-administered agents. For example, with the form of injectable agents, administration may be systemically or locally via intravenous injection, intramuscular injection, intraperitoneal injection, subcutaneous injection, or intratumoral injection. The form of percutaneously-administered agents include, for example, agents called liniments and external medicines. The external medicines include, for example, solid agents, solutions, sprays, ointments, creams, and gels.

The administration method can be appropriately determined depending on age, weight, sex, and symptoms of a patient. The dose of a pharmaceutical composition comprising at least one of the active ingredients in a medicament according to the present invention can be selected within a range of, for example, 0.0001 mg to 1000 mg per kg of body weight per dose, as the amount of each active ingredient. Alternatively, the dose of each active ingredient can be selected within a range of, for example, 0.001 to 100000 mg per patient's body or 1 mg to 30 mg per kg of patient's body weight; however, it is not necessarily limited thereto. The dose and the administration method are changed depending on the patient age, weight, gender, and symptoms. However, persons skilled in the art can appropriately select the dose and the method.

### <Administration method>

Treatment and/or prevention of cancer with a medicament according to the present invention includes various modes, in addition to administration as a medicament mentioned above. For example, respective active ingredients in a medicament of the present invention can be administered simultaneously, in parallel, or individually in a staggered manner. As a specific example, the second, third, and fourth active ingredients can be administered within a time interval up to approximately 3 weeks after administration of the first active ingredient, i.e., the active ingredients can be administered from immediately after up to approximately 3 weeks after administration of the first active ingredient. These administrations may be carried out subsequently to a surgical procedure, or a surgical procedure may be carried out between the administration of the first drug and the administrations of the second, third, and fourth drugs. In addition, the medicament for treatment and/or prevention of cancer of the present invention may be administered according to a plurality of administration cycles. For example, in the case of carrying out simultaneous administration of respective active ingredients in a medicament for treatment and/or prevention of cancer of the present invention, a pharmaceutical composition comprising the active ingredients of the present invention is administered once per approximately 2 days to approximately 3 weeks as one cycle. Then, this treatment cycle may be repeated, if necessary, according to the judgment of a physician in charge. Likewise, in the case of scheduling a formulation in a staggered manner, respective administration periods of individual agents are adjusted so as to span the same period. The interval between cycles can vary from 0 to 2 months. Doses of the respective active ingredients in a medicament for treatment and/or prevention of cancer of the present invention can be set in the same way as in the doses of the respective active ingredients in the pharmaceutical composition.

### <Pharmaceutical kit>

A medicament according to the present invention may be in the form of a pharmaceutical kit. The pharmaceutical kit may be a package for using active ingredients in the form of separate pharmaceutical compositions (formulations) in a method for treating and/or preventing cancer, and may comprise an instruction for administering each of the active ingredients. The respective active ingredients in the pharmaceutical compositions for treatment and/or prevention of cancer contained in the pharmaceutical kit can be in the form of pharmaceutical compositions each formulated as described above such that the active ingredients can be administered together or separately. Further, the pharmaceutical kit comprises active ingredients in amounts sufficient for one or more doses such that the active ingredients can be administered according to the administration method described above.

### <Method for treatment and/or prevention>

On the basis of the contents specifically described above, the present invention provides a method for treating and/or preventing cancer, comprising administering, to a subject (patient), the medicament of the present invention or a medicament of the present invention. For example, the present invention further provides a method for treating and/or preventing cancer, comprising administering the medicament or the like of the present invention as described above to a subject (patient) having cancer or suspected of having cancer. In the method of the present invention, another antitumor agent (a known antitumor agent or the like) in addition to the medicament of the present invention may be administered to a subject (patient) (provided that a pyrimidine-based drug is not administered). In this embodiment, for example, drugs serving as active ingredients of a medicament according to the present invention are administered simultaneously or separately to the subject (patient).

The present invention also provides a medicament for use in combination with another drug in said method for treating and/or preventing cancer, wherein the medicament comprises, as active ingredients, one or more selected from the group consisting of an antibody or a fragment thereof having an immunological reactivity with CAPRIN-1 protein, an anthracycline-based drug, a platinum formulation, and an angiogenesis inhibitor (but not comprising a pyrimidine-based drug as an active ingredient). When the medicament comprises a plurality of active ingredients, each ingredient can be contained together or separately.

### <Drug Efficacy Increasing Agent>

On the basis of the contents specifically described above, the present invention provides a drug efficacy increasing agent comprising an antibody or a fragment thereof having immunological reactivity with the CAPRIN-1 protein, or an anthracycline-based drug and a platinum drug, which are the active ingredients of the medicament according to the present invention. The medicament whose drug efficacy is enhanced with the drug efficacy increasing agent comprises an active ingredient that is not contained in the drug efficacy increasing agent, and one or both of the drug efficacy increasing agent and the medicament do not comprise a pyrimidine-based drug as an active ingredient. The drug efficacy increasing agent and/or the medicament whose efficacy is enhanced can further comprise an angiogenesis inhibitor as the active ingredient. Specifically, when the drug efficacy increasing agent comprises an anti-CAPRIN-1 antibody, for example, the medicament whose efficacy is enhanced can comprise an anthracycline-based drug and a platinum formulation, or an anthracycline-based drug, a platinum formulation, and an angiogenesis inhibitor. When the drug efficacy increasing agent comprises an anthracycline-based drug and a platinum formulation, or an anthracycline-based drug, a platinum formulation, and an angiogenesis inhibitor, the medicament can comprise an anti-CAPRIN-1 antibody.

For example, when the drug efficacy increasing agent and/or the medicament whose efficacy is enhanced comprises a plurality of active ingredients, the drug and/or the medicament can comprise each ingredient together or separately. Preferably, the method for treating and/or preventing cancer according to the present invention can be carried out by comprising an effective amount of an anti-CAPRIN-1 antibody, or an anthracycline-based drug and a platinum formulation in at least one of the drug efficacy increasing agent and the medicament whose efficacy is enhanced, or using the drug efficacy increasing agent and the medicament whose efficacy is enhanced. In this case, the drug efficacy increasing agent and the medicament whose efficacy is enhanced may comprise an active ingredient that is not contained in an effective amount in either of them or comprise the same type of active ingredient in both.

### Examples

The present invention is hereafter described in detail with reference to the following examples, although the scope of the present invention is not limited thereto.

### (Example 1) Production of anti-CAPRIN-1 antibody

As anti-CAPRIN-1 antibodies having an immunological reactivity with CAPRIN-1 protein used in the present invention, antibodies were produced as described below for use.

### (Polyclonal antibody)

1 mg of a human CAPRIN-1 recombinant protein (SEQ ID NO: 2) produced according to Example 3 of WO2010/016526 was mixed with an incomplete Freund's adjuvant (IFA) solution in an amount equivalent to the recombinant protein. The mixture was subcutaneously administered to a rabbit four times every 2 weeks. Subsequently, blood was collected, so that an antiserum containing a polyclonal antibody was obtained. Furthermore, the antiserum was purified using a protein G carrier (GE Healthcare Bio-Sciences) and replaced with PBS(-) and then a polyclonal antibody against CAPRIN-1 protein (anti-CAPRIN-1 polyclonal antibody #1) was obtained.

### (Monoclonal antibody)

100 µg of a human CAPRIN-1 recombinant protein produced according to Example 3 of WO2010/016526 was mixed with an MPL+TDM adjuvant (Sigma) in an amount equivalent to that of the antigen protein. The mixture was used as an antigen solution per mouse. The antigen solution was administered intraperitoneally to 6-week-old Balb/c mice (Japan SLC Inc.) and then further administered 3 times and 24 times every week for completion of immunization. A spleen was removed on day 3 from the final immunization and then ground between two sterilized glass slides. Spleen cells were obtained by repeating the following procedure three times: washing with PBS(-) (manufactured by Nissui Pharmaceutical Co., Ltd.), centrifuging at 1500 rpm for 10 minutes, and removing the supernatant. The obtained spleen cells were mixed with mouse myeloma cell SP2/0 (purchased from ATCC) at a ratio of 10:1. The PEG solution prepared by mixing 200 µl of RPMI1640 medium containing 10% FBS heated at 37°C and 800 µl of PEG1500 (Boehringer) was added to the cells. The solution was incubated for 5 minutes for cell fusion. Centrifugation was performed at 1700 rpm for 5 minutes, and the supernatants were removed. Cells were suspended in 150 ml of RPMI1640 medium (HAT selective medium) containing 15% FBS, to which 2% equivalent of HAT solution (Gibco) had been added and then seeded onto fifteen 96-well plates (Nunc) at 100 µl per well. Cells were cultured for 7 days under conditions of 37°C and 5% CO₂, so that hybridomas resulting from fusion of spleen cells to myeloma cells were obtained. Hybridomas were selected using binding affinity to CAPRIN-1 protein of the antibody produced by the prepared hybridomas as an indicator. The CAPRIN-1 protein solution (1 µg/ml) was added at 100 µl per well of 96-well plates and then incubated at 4°C for 18 hours. After each well was washed three times with PBS-T, 0.5% Bovine Serum Albumin (BSA) solution (Sigma) was added at 400 µl per well, and then the plates were incubated at room temperature for 3 hours. The solution was removed and then each well was washed three times with 400 µl of PBS-T. Each culture supernatant of the hybridomas obtained above was added at 100 µl per well and then incubated at room temperature for 2 hours. After each well was washed three times with PBS-T, an HRP-labeled anti-mouse IgG (H+L) antibody (Invitrogen) diluted 5000-fold with PBS was added at 100 µl per well and then incubated at room temperature for 1 hour. After each well was washed three times with PBS-T, a TMB substrate solution (Thermo) was added at 100 µl per well and then incubated for 15-30 minutes, so that a color reaction was performed. After color development, 1 N sulfuric acid was added at 100 µl per well to stop the reaction. Absorbance at 450 nm and absorbance at 595 nm were measured using an absorption spectrometer. As a result, a plurality of hybridomas producing antibodies with high absorbances were selected. The selected hybridomas were added at 0.5 hybridomas per well of 96-well plates and then cultured. After 1 week, hybridomas forming single colonies in wells were observed. Cells in these wells were further cultured. Hybridomas were selected using binding affinity to CAPRIN-1 protein of the antibody produced by cloned hybridomas as an indicator. The CAPRIN-1 protein solution (1 µg/ml) was added at 100 µl per well of 96-well plates and then incubated at 4°C for 18 hours. Each well was washed three times with PBS-T, and a 0.5% BSA solution was added at 400 µl per well, and then incubated at room temperature for 3 hours. The solution was removed and then each well was washed three times with 400 µl of PBS-T. Each culture supernatant of the hybridomas obtained above was added at 100 µl per well and then incubated at room temperature for 2 hours. Each well was washed three times with PBS-T, an HRP-labeled anti-mouse IgG (H+L) antibody (Invitrogen) diluted 5000-fold with PBS was added at 100 µl per well and then incubated at room temperature for 1 hour. Each well was washed three times with PBS-T, a TMB substrate solution (Thermo) was added at 100 µl per well and then incubated for 15-30 minutes, so that a color reaction was performed. After color development, 1 N sulfuric acid was added at 100 µl per well to stop the reaction. Absorbance at 450 nm and absorbance at 595 nm were measured using an absorption spectrometer. As a result, a plurality of mouse monoclonal antibodies exerting reactivity with CAPRIN-1 protein were obtained.

Reactivity of each monoclonal antibody with human cancer cells confirmed to express CAPRIN-1 protein on cell membrane surfaces was further confirmed by flow cytometry. A mouse IgG control antibody exhibiting no reactivity with the cancer cells was used as a negative control. As a result of confirmation, several monoclonal antibodies were obtained which had stronger fluorescence intensity against the cancer cells than that of the mouse IgG control antibody and reacted strongly with the cell membrane surfaces of the cancer cells expressing CAPRIN-1 on the cell membrane surfaces. Among them, a monoclonal antibody against CAPRIN-1 described in WO2013/125630, which was an antibody in the above-mentioned (ab) comprising the amino acid sequence of a heavy-chain variable region shown by SEQ ID NO: 114 and the amino acid sequence of a light-chain variable region shown by SEQ ID NO: 115, was selected as a monoclonal antibody exhibiting reactivity with CAPRIN-1 protein.

CDR1 to CDR3 of the heavy-chain variable region of the antibody selected were identified. A nucleotide sequence was designed such that a heavy-chain variable region comprising a human antibody sequence as a framework region. This nucleotide sequence was inserted to a vector for mammalian expression having an insert of a human IgG1 heavy-chain constant region. Likewise, CDR1 to CDR3 of the light-chain variable region were identified. A nucleotide sequence was designed such that a light-chain variable region comprising a human antibody sequence as a framework region. This nucleotide sequence was inserted to a vector for mammalian expression having an insert of a human IgG1 light-chain constant region. These two recombinant expression vectors were introduced to mammalian cells according to a general method and then a culture supernatant containing humanized monoclonal antibody #1 (humanized antibody #1) against CAPRIN-1.

The obtained culture supernatant containing the obtained humanized anti-CAPRIN-1 monoclonal antibody #1 was purified using Hitrap Protein A Sepharose FF (GE Healthcare Bio-Sciences) according to a general method, replaced with PBS(-), and filtered through a 0.22 µm filter (Millipore) for preparation of the filtrate.

The specific reactivity of the anti-CAPRIN-1 antibody to CAPRIN-1 protein was detected and confirmed by ELISA using CAPRIN-1 protein immobilized on a plate.

The reactivity of the anti-CAPRIN-1 antibody with cancer cells without permeation treatment of cell membranes was examined by flow cytometry to confirm that a portion of CAPRIN-1 was expressed on cell membrane surfaces of cancer cells as shown by Examples given below.

It was confirmed by flow cytometry that, against all of breast cancer cells (BT-474), colon cancer cells (HT-29), lung cancer cells (QG56 and H1650), gastric cancer cells (NCI-N87), uterus cancer cells (HEC-1-A), prostate cancer cells (22Rv1), pancreatic cancer cells (Panc10.5), liver cancer cells (Hep3B), ovarian cancer cells (SKOV3), kidney cancer cells (Caki-2), brain tumor cells (U-87MG), urinary bladder cancer cells (T24), esophageal cancer cells (OE33), leukemia cells (OCI-AML5), lymphoma cells (Ramos), gallbladder cancer cells (TGBC14TKB), fibrosarcoma cells (HT-1080), and melanoma cells (G-361), which are human cancer cells confirmed to express CAPRIN-1 gene; and mouse kidney cancer cells (Renca) and mouse breast cancer cells (4T1) confirmed to express CAPRIN-1 gene, the humanized antibody #1 had stronger fluorescence intensity than that of a human IgG control antibody and rabbit IgG antibody serving as negative controls exhibiting no reactivity with the cancer cells, and reacted strongly with the cell membrane surfaces of the cancer cells expressing CAPRIN-1.

Similarly, it was confirmed that each of those anti-CAPRIN-1 antibodies described in (a) to (aa) and (ac) to (al) above which are described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176, and WO2015/020212 also reacted strongly with the cell membrane surface of the cancer in the same manner.

### (Example 2) Antitumor effect of a combined use of anti-CAPRIN-1 antibody and doxorubicin hydrochloride liposome formulation (PLD)/carboplatin (CBDCA) therapy in mouse model bearing cancer of human cancer cell

An *in vivo* antitumor effect in a cancer-bearing mouse by a combined use of the anti-CAPRIN-1 antibody (anti-CAPRIN-1 humanized antibody #1) produced in Example 1 and doxorubicin hydrochloride liposome formulation (PLD)/carboplatin (CBDCA) therapy was evaluated.

Specifically, the antitumor effect of the combined use of an anti-CAPRIN-1 antibody and a doxorubicin hydrochloride liposome formulation (PLD)/carboplatin (CBDCA) therapy according to the present invention was studied using NOD-SCID mice in which human-derived cancer cells expressing CAPRIN-1 protein were subcutaneously transplanted. Human breast cancer cells BT-474 were subcutaneously transplanted at 2 × 10⁷ cells per mouse as a mixture with Matrigel (Corning) and allowed to grow until a tumor became approximately 100 to 200 mm³ to prepare cancer-bearing mice. The BT-474 is a cancer cell that expresses CAPRIN-1 protein on cell membrane surfaces, and for which the anti-CAPRIN-1 antibodies produced in Example 1 were confirmed to react with a portion of CAPRIN-1 present on the cell membrane surfaces. Each anti-CAPRIN-1 antibody produced in Example 1 was administered at 10 mg/kg once a week, four times in total, via the tail veins of five cancer-bearing mice described above. A doxorubicin hydrochloride liposome formulation (PLD) and carboplatin (CBDCA) were administered intravenously at 1 mg/kg and intraperitoneally at 15 mg/kg, respectively, at the same time as an anti-CAPRIN-1 antibody was administered. A doxorubicin hydrochloride liposome formulation (PLD) and carboplatin (CBDCA) were each administered once a week, four times in total (a group treated with a combination of anti-CAPRIN-1 antibody/PLD/CBDCA).

For a comparative control group, the same dose of the same anti-CAPRIN-1 antibody as above was administered once a week to cancer-bearing mice (anti-CAPRIN-1 antibody-treated group). For another comparative control group, only doxorubicin hydrochloride liposome formulation (PLD)/carboplatin (CBDCA) (a group treated with a combination of PLD/CBDCA) was administered to other cancer-bearing mouse individuals at the same administration intervals. Cancer-bearing mice in a predetermined Vehicle treated group were used as negative controls. After the start of administration, the sizes of cancers in the cancer-bearing mice were measured over time using calipers. The tumor volume of each individual was calculated according to a standard method using a calculation formula: (Length of the major axis of the tumor) × (Length of the minor axis of the tumor)² × 0.5, and the average of the treated group was calculated.

As the result of the evaluation, the tumor volumes of the comparative control groups were as follows: the tumor volumes of the anti-CAPRIN-1 antibody-treated group and the group treated with a combination of PLD/CBDCA were both 45% or more, but the tumor volume of the group treated with a combination of anti-CAPRIN-1 antibody/PLD/CBDCA was 25% or less, in approximately one week after the termination of the administration of the drug (on day 45 after bearing cancer), assuming that the tumor volume of the negative control was 100%. Thus, the group treated with a combination of anti-CAPRIN-1 antibody/PLD/CBDCA exhibited a remarkable antitumor effect as compared with the group treated with a combination of

### PLD/CBDCA.

Similarly, it was confirmed that each of those anti-CAPRIN-1 antibodies described in (a) to (aa) and (ac) to (al) above which are described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176, and WO2015/020212 had a remarkably stronger antitumor effect when used in combination with the PLD/CBDCA therapy than the anti-CAPRIN-1 antibody alone or the PLD/CBDCA therapy.

The results of this evaluation have demonstrated that each of the anti-CAPRIN-1 antibodies had a remarkably stronger antitumor effect when used in combination with the doxorubicin hydrochloride liposome formulation (PLD)/carboplatin (CBDCA) therapy than the anti-CAPRIN-1 antibody alone or the doxorubicin hydrochloride liposome formulation (PLD)/carboplatin (CBDCA) therapy.

### (Example 3) Antitumor effect of a combined use of anti-CAPRIN-1 antibody and doxorubicin hydrochloride liposome formulation (PLD)/carboplatin (CBDCA)/bevacizumab (hereinafter referred to as BVZ) therapy in mouse model bearing cancer of human cancer cell

An *in vivo* antitumor effect in a cancer-bearing mouse by a combined use of the anti-CAPRIN-1 antibody (anti-CAPRIN-1 humanized antibody #1) produced in Example 1 and a doxorubicin hydrochloride liposome formulation (PLD)/carboplatin (CBDCA)/bevacizumab (BVZ) therapy was evaluated.

Specifically, the antitumor effect of the combined use of an anti-CAPRIN-1 antibody and a PLD/CBDCA/BVZ therapy according to the present invention was studied using NOD-SCID mice in which human-derived cancer cells expressing CAPRIN-1 protein were subcutaneously transplanted. Human breast cancer cells BT-474 were subcutaneously transplanted at 2 × 10⁷ cells per mouse as a mixture with Matrigel (Corning) and allowed to grow until a tumor became approximately 100 to 200 mm³ to prepare cancer-bearing mice. The BT-474 is a cancer cell that expresses CAPRIN-1 protein on cell membrane surfaces, and for which the anti-CAPRIN-1 antibodies produced in Example 1 were confirmed to react with a portion of CAPRIN-1 present on the cell membrane surfaces. Each anti-CAPRIN-1 antibody produced in Example 1 was administered at 10 mg/kg once a week, four times in total, via the tail veins of five cancer-bearing mice described above. To each of the same mice, PLD, CBDCA, and BVZ were administered intravenously at 1 mg/kg, intraperitoneally at 15 mg/kg, and intraperitoneally at 1.25 mg/kg, respectively, at the same time as an anti-CAPRIN-1 antibody was administered. PLD, CBDCA, and BVZ were each administered once a week four times in total (a group treated with a combination of anti-CAPRIN-1 antibody/PLD/CBDCA/BVZ).

For a comparative control group, the same dose of the same anti-CAPRIN-1 antibody as above was administered once a week to cancer-bearing mice (an anti-CAPRIN-1 antibody-treated group). For another comparative control group, only PLD/CBDCA/BVZ (a group treated with a combination of PLD/CBDCA/BVZ) was administered to other cancer-bearing mouse individuals at the same administration intervals. Cancer-bearing mice in a predetermined Vehicle treated group were used as negative controls. After the start of administration, the sizes of cancers in the cancer-bearing mice were measured over time using calipers. The tumor volume of each individual was calculated according to a standard method using a calculation formula: (Length of the major axis of the tumor) × (Length of the minor axis of the tumor)² × 0.5, and the average of the treated group was calculated.

As the result of the evaluation, the tumor volumes of the comparative control groups were as follows: the tumor volumes of the anti-CAPRIN-1 antibody-treated group and the group treated with a combination of PLD/CBDCA/BVZ were both 45% or more, but the tumor volume of the group treated with a combination of anti-CAPRIN-1 antibody/PLD/CBDCA/BVZ was 15% or less, in approximately one week after the termination of the administration of the drug (on day 45 after bearing cancer), assuming that the tumor volume of the negative control was 100%. Thus, the group treated with a combination of anti-CAPRIN-1 antibody/PLD/CBDCA/BVZ exhibited a remarkable antitumor effect as compared with the group treated with a combination of PLD/CBDCA/BVZ.

Similarly, it was confirmed that each of those anti-CAPRIN-1 antibodies described in (a) to (aa) and (ac) to (al) above which are described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176, and WO2015/020212 had a remarkably stronger antitumor effect when used in combination with the PLD/CBDCA/BVZ therapy than the anti-CAPRIN-1 antibody alone or the PLD/CBDCA/BVZ therapy.

The results of this evaluation have demonstrated that each of the anti-CAPRIN-1 antibodies had a remarkably stronger antitumor effect when used in combination with the doxorubicin hydrochloride liposome formulation (PLD)/carboplatin (CBDCA)/bevacizumab (BVZ) therapy than the anti-CAPRIN-1 antibody alone or the doxorubicin hydrochloride liposome formulation (PLD)/carboplatin (CBDCA)/bevacizumab (BVZ) therapy.

In addition, as a result of the evaluation of Example 2 and Example 3, the antitumor effect of the doxorubicin hydrochloride liposome formulation (PLD)/carboplatin (CBDCA)/bevacizumab (BVZ) therapy was similar to that of the doxorubicin hydrochloride liposome formulation (PLD)/carboplatin (CBDCA) therapy, and no significant enhancement of the antitumor effect was observed by using bevacizumab (BVZ) in combination. On the other hand, the antitumor effect of the combined use of the anti-CAPRIN-1 antibody and the doxorubicin hydrochloride liposome formulation (PLD)/carboplatin (CBDCA)/bevacizumab (BVZ) therapy was superior to the antitumor effect of the anti-CAPRIN-1 antibody and the doxorubicin hydrochloride liposome formulation (PLD)/carboplatin (CBDCA) therapy, and thus, the enhanced antitumor effect was observed with the combination of bevacizumab (BVZ).

These results have revealed that the enhanced antitumor effect of the combined use of the anti-CAPRIN-1 antibody and the doxorubicin hydrochloride liposome (PLD)/carboplatin (CBDCA) therapy or the doxorubicin hydrochloride liposome (PLD)/carboplatin (CBDCA)/bevacizumab (BVZ) therapy was an unexpected synergistic effect of each drug.

All publications, patents, and patent applications cited herein are hereby incorporated by reference in their entirety.

## Claims

1. A medicament for treatment and/or prevention of cancer, comprising: an antibody or a fragment thereof having an immunological reactivity with CAPRIN-1 protein; an anthracycline-based drug; and a platinum formulation, together or separately in combination (excluding a pyrimidine-based drug).

2. The medicament according to claim 1, further comprising an angiogenesis inhibitor together or separately in combination.

3. The medicament according to claim 1 or 2, wherein the anthracycline-based drug is doxorubicin or a doxorubicin hydrochloride liposome formulation (PLD).

4. The medicament according to any one of claims 1 to 3, wherein the platinum formulation is carboplatin, cisplatin, oxaliplatin, nedaplatin, and/or miriplatin.

5. The medicament according to any one of claims 2 to 4, wherein the angiogenesis inhibitor is bevacizumab.

6. The medicament according to any one of claims 1 to 5, wherein the antibody or the fragment thereof has an immunological reactivity with CAPRIN-1 protein having an amino acid sequence shown by any one of the even numbered SEQ ID NOs among SEQ ID NOs: 2 to 30, or an amino acid sequence having 80% or more sequence identity with the amino acid sequence.

7. The medicament according to any one of claims 1 to 6, wherein the antibody or the fragment thereof has an immunological reactivity with an extracellular region of CAPRIN-1 protein present on a cancer cell surface.

8. The medicament according to any one of claims 1 to 7, wherein the antibody or the fragment thereof has an immunological reactivity with a partial polypeptide of CAPRIN-1 protein, the partial polypeptide having an amino acid sequence represented by any one of SEQ ID NOs: 31 to 35, 296 to 299, 308 and 309, or an amino acid sequence having 80% or more sequence identity with the amino acid sequence.

9. The medicament according to any one of claims 1 to 8, wherein the antibody is a monoclonal antibody or a polyclonal antibody.

10. The medicament according to any one of claims 1 to 9, wherein the antibody or the fragment thereof is any one of the following (A) to (M):
(A) an antibody or a fragment thereof comprising a heavy-chain variable region comprising complementarity determining regions of SEQ ID NOs: 36, 37 and 38 (CDR1, CDR2 and CDR3, respectively) and a light-chain variable region comprising complementarity determining regions of SEQ ID NOs: 40, 41 and 42 (CDR1, CDR2 and CDR3, respectively), and having an immunological reactivity with CAPRIN-1 protein;
(B) an antibody or a fragment thereof comprising a heavy-chain variable region comprising complementarity determining regions of SEQ ID NOs: 44, 45 and 46 (CDR1, CDR2 and CDR3, respectively) and a light-chain variable region comprising complementarity determining regions of SEQ ID NOs: 48, 49 and 50 (CDR1, CDR2 and CDR3, respectively), and having an immunological reactivity with CAPRIN-1 protein;
(C) an antibody or a fragment thereof comprising a heavy-chain variable region comprising complementarity determining regions of SEQ ID NOs: 52, 53 and 54 (CDR1, CDR2 and CDR3, respectively) and a light-chain variable region comprising complementarity determining regions of SEQ ID NOs: 56, 57 and 58 (CDR1, CDR2 and CDR3, respectively), and having an immunological reactivity with CAPRIN-1 protein;
(D) an antibody or a fragment thereof comprising a heavy-chain variable region comprising complementarity determining regions of SEQ ID NOs: 60, 61 and 62 (CDR1, CDR2 and CDR3, respectively) and a light-chain variable region comprising complementarity determining regions of SEQ ID NOs: 64, 65 and 66 (CDR1, CDR2 and CDR3, respectively), and having an immunological reactivity with CAPRIN-1 protein;
(E) an antibody or a fragment thereof comprising a heavy-chain variable region comprising complementarity determining regions of SEQ ID NOs: 170, 171 and 172 (CDR1, CDR2 and CDR3, respectively) and a light-chain variable region comprising complementarity determining regions of SEQ ID NOs: 173, 174 and 175 (CDR1, CDR2 and CDR3, respectively), and having an immunological reactivity with CAPRIN-1 protein;
(F) an antibody or a fragment thereof comprising a heavy-chain variable region comprising complementarity determining regions of SEQ ID NOs: 176, 177 and 178 (CDR1, CDR2 and CDR3, respectively) and a light-chain variable region comprising complementarity determining regions of SEQ ID NOs: 179, 180 and 181 (CDR1, CDR2 and CDR3, respectively), and having an immunological reactivity with CAPRIN-1 protein;
(G) an antibody or a fragment thereof comprising a heavy-chain variable region comprising complementarity determining regions of SEQ ID NOs: 182, 183 and 184 (CDR1, CDR2 and CDR3, respectively) and a light-chain variable region comprising complementarity determining regions of SEQ ID NOs: 185, 186 and 187 (CDR1, CDR2 and CDR3, respectively), and having an immunological reactivity with CAPRIN-1 protein;
(H) an antibody or a fragment thereof comprising a heavy-chain variable region comprising complementarity determining regions of SEQ ID NOs: 188, 189 and 190 (CDR1, CDR2 and CDR3, respectively) and a light-chain variable region comprising complementarity determining regions of SEQ ID NOs: 191, 192 and 193 (CDR1, CDR2 and CDR3, respectively), and having an immunological reactivity with CAPRIN-1 protein;
(I) an antibody or a fragment thereof comprising a heavy-chain variable region comprising complementarity determining regions of SEQ ID NOs: 146, 147 and 148 (CDR1, CDR2 and CDR3, respectively) and a light-chain variable region comprising complementarity determining regions of SEQ ID NOs: 149, 150 and 151 (CDR1, CDR2 and CDR3, respectively), and having an immunological reactivity with CAPRIN-1 protein;
(J) an antibody or a fragment thereof comprising a heavy-chain variable region comprising complementarity determining regions of SEQ ID NOs: 272, 273 and 274 (CDR1, CDR2 and CDR3, respectively) and a light-chain variable region comprising complementarity determining regions of SEQ ID NOs: 275, 276 and 277 (CDR1, CDR2 and CDR3, respectively), and having an immunological reactivity with CAPRIN-1 protein;
(K) an antibody or a fragment thereof comprising a heavy-chain variable region comprising complementarity determining regions of SEQ ID NOs: 290, 291 and 292 (CDR1, CDR2 and CDR3, respectively) and a light-chain variable region comprising complementarity determining regions of SEQ ID NOs: 293, 294 and 295 (CDR1, CDR2 and CDR3, respectively), and having an immunological reactivity with CAPRIN-1 protein;
(L) an antibody or a fragment thereof comprising a heavy-chain variable region comprising complementarity determining regions of SEQ ID NOs: 300, 301 and 302 (CDR1, CDR2 and CDR3, respectively) and a light-chain variable region comprising complementarity determining regions of SEQ ID NOs: 304, 305 and 306 (CDR1, CDR2 and CDR3, respectively), and having an immunological reactivity with CAPRIN-1 protein; and
(M) an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein.

11. The medicament according to any one of claims 1 to 10, wherein the antibody or the fragment thereof is any one of the following (a) to (al):
(a) an antibody or a fragment thereof comprising a heavy-chain variable region comprising the amino acid sequence of SEQ ID NO: 39 and a light-chain variable region comprising the amino acid sequence of SEQ ID NO: 43;
(b) an antibody or a fragment thereof comprising a heavy-chain variable region comprising the amino acid sequence of SEQ ID NO: 47 and a light-chain variable region comprising the amino acid sequence of SEQ ID NO: 51;
(c) an antibody or a fragment thereof comprising a heavy-chain variable region comprising the amino acid sequence of SEQ ID NO: 55 and a light-chain variable region comprising the amino acid sequence of SEQ ID NO: 59;
(d) an antibody or a fragment thereof comprising a heavy-chain variable region comprising the amino acid sequence of SEQ ID NO: 63 and a light-chain variable region comprising the amino acid sequence of SEQ ID NO: 67;
(e) an antibody or a fragment thereof comprising a heavy-chain variable region comprising the amino acid sequence of SEQ ID NO: 68 and a light-chain variable region comprising the amino acid sequence of SEQ ID NO: 69;
(f) an antibody or a fragment thereof comprising a heavy-chain variable region comprising the amino acid sequence of SEQ ID NO: 70 and a light-chain variable region comprising the amino acid sequence of SEQ ID NO: 71;
(g) an antibody or a fragment thereof comprising a heavy-chain variable region comprising the amino acid sequence of SEQ ID NO: 72 and a light-chain variable region comprising the amino acid sequence of SEQ ID NO: 73;
(h) an antibody or a fragment thereof comprising a heavy-chain variable region comprising the amino acid sequence of SEQ ID NO: 74 and a light-chain variable region comprising the amino acid sequence of SEQ ID NO: 75;
(i) an antibody or a fragment thereof comprising a heavy-chain variable region comprising the amino acid sequence of SEQ ID NO: 76 and a light-chain variable region comprising the amino acid sequence of SEQ ID NO: 77;
(j) an antibody or a fragment thereof comprising a heavy-chain variable region comprising the amino acid sequence of SEQ ID NO: 78 and a light-chain variable region comprising the amino acid sequence of SEQ ID NO: 79;
(k) an antibody or a fragment thereof comprising a heavy-chain variable region comprising the amino acid sequence of SEQ ID NO: 80 and a light-chain variable region comprising the amino acid sequence of SEQ ID NO: 81;
(l) an antibody or a fragment thereof comprising a heavy-chain variable region comprising the amino acid sequence of SEQ ID NO: 82 and a light-chain variable region comprising the amino acid sequence of SEQ ID NO: 83;
(m) an antibody or a fragment thereof comprising a heavy-chain variable region comprising the amino acid sequence of SEQ ID NO: 84 and a light-chain variable region comprising the amino acid sequence of SEQ ID NO: 85;
(n) an antibody or a fragment thereof comprising a heavy-chain variable region comprising the amino acid sequence of SEQ ID NO: 86 and a light-chain variable region comprising the amino acid sequence of SEQ ID NO: 87;
(o) an antibody or a fragment thereof comprising a heavy-chain variable region comprising the amino acid sequence of SEQ ID NO: 88 and a light-chain variable region comprising the amino acid sequence of SEQ ID NO: 89;
(p) an antibody or a fragment thereof comprising a heavy-chain variable region comprising the amino acid sequence of SEQ ID NO: 90 and a light-chain variable region comprising the amino acid sequence of SEQ ID NO: 91;
(q) an antibody or a fragment thereof comprising a heavy-chain variable region comprising the amino acid sequence of SEQ ID NO: 92 and a light-chain variable region comprising the amino acid sequence of SEQ ID NO: 93;
(r) an antibody or a fragment thereof comprising a heavy-chain variable region comprising the amino acid sequence of SEQ ID NO: 94 and a light-chain variable region comprising the amino acid sequence of SEQ ID NO: 95;
(s) an antibody or a fragment thereof comprising a heavy-chain variable region comprising the amino acid sequence of SEQ ID NO: 96 and a light-chain variable region comprising the amino acid sequence of SEQ ID NO: 97;
(t) an antibody or a fragment thereof comprising a heavy-chain variable region comprising the amino acid sequence of SEQ ID NO: 98 and a light-chain variable region comprising the amino acid sequence of SEQ ID NO: 99;
(u) an antibody or a fragment thereof comprising a heavy-chain variable region comprising the amino acid sequence of SEQ ID NO: 100 and a light-chain variable region comprising the amino acid sequence of SEQ ID NO: 101;
(v) an antibody or a fragment thereof comprising a heavy-chain variable region comprising the amino acid sequence of SEQ ID NO: 102 and a light-chain variable region comprising the amino acid sequence of SEQ ID NO: 103;
(w) an antibody or a fragment thereof comprising a heavy-chain variable region comprising the amino acid sequence of SEQ ID NO: 104 and a light-chain variable region comprising the amino acid sequence of SEQ ID NO: 105;
(x) an antibody or a fragment thereof comprising a heavy-chain variable region comprising the amino acid sequence of SEQ ID NO: 106 and a light-chain variable region comprising the amino acid sequence of SEQ ID NO: 107;
(y) an antibody or a fragment thereof comprising a heavy-chain variable region comprising the amino acid sequence of SEQ ID NO: 108 and a light-chain variable region comprising the amino acid sequence of SEQ ID NO: 109;
(z) an antibody or a fragment thereof comprising a heavy-chain variable region comprising the amino acid sequence of SEQ ID NO: 110 and a light-chain variable region comprising the amino acid sequence of SEQ ID NO: 111;
(aa) an antibody or a fragment thereof comprising a heavy-chain variable region comprising the amino acid sequence of SEQ ID NO: 112 and a light-chain variable region comprising the amino acid sequence of SEQ ID NO: 113;
(ab) an antibody or a fragment thereof comprising a heavy-chain variable region comprising the amino acid sequence of SEQ ID NO: 114 and a light-chain variable region comprising the amino acid sequence of SEQ ID NO: 115;
(ac) an antibody or a fragment thereof comprising a heavy-chain variable region comprising the amino acid sequence of SEQ ID NO: 116 and a light-chain variable region comprising the amino acid sequence of SEQ ID NO: 117;
(ad) an antibody or a fragment thereof comprising a heavy-chain variable region comprising the amino acid sequence of SEQ ID NO: 118 and a light-chain variable region comprising the amino acid sequence of SEQ ID NO: 119;
(ae) an antibody or a fragment thereof comprising a heavy-chain variable region comprising the amino acid sequence of SEQ ID NO: 120 and a light-chain variable region comprising the amino acid sequence of SEQ ID NO: 121;
(af) an antibody or a fragment thereof comprising a heavy-chain variable region comprising the amino acid sequence of SEQ ID NO: 122 and a light-chain variable region comprising the amino acid sequence of SEQ ID NO: 123;
(ag) an antibody or a fragment thereof comprising a heavy-chain variable region comprising the amino acid sequence of SEQ ID NO: 124 and a light-chain variable region comprising the amino acid sequence of SEQ ID NO: 125;
(ah) an antibody or a fragment thereof comprising a heavy-chain variable region comprising the amino acid sequence of SEQ ID NO: 126 and a light-chain variable region comprising the amino acid sequence of SEQ ID NO: 127;
(ai) an antibody or a fragment thereof comprising a heavy-chain variable region comprising the amino acid sequence of SEQ ID NO: 128 and a light-chain variable region comprising the amino acid sequence of SEQ ID NO: 129;
(aj) an antibody or a fragment thereof comprising a heavy-chain variable region comprising the amino acid sequence of SEQ ID NO: 130 and a light-chain variable region comprising the amino acid sequence of SEQ ID NO: 131;
(ak) an antibody or a fragment thereof comprising a heavy-chain variable region comprising the amino acid sequence of SEQ ID NO: 132 and a light-chain variable region comprising the amino acid sequence of SEQ ID NO: 133; and
(al) an antibody or a fragment thereof comprising a heavy-chain variable region comprising the amino acid sequence of SEQ ID NO: 303 and a light-chain variable region comprising the amino acid sequence of SEQ ID NO: 307.

12. The medicament according to any one of claims 1 to 11, wherein the antibody is a human antibody, a humanized antibody, a chimeric antibody or a single chain antibody.

13. The medicament according to any one of claims 1 to 12, wherein the cancer is a cancer expressing CAPRIN-1 protein on a cell membrane surface.

14. The medicament according to any one of claims 1 to 13, wherein the cancer is ovarian cancer, bile duct cancer, breast cancer, kidney cancer, pancreatic cancer, colon cancer, melanoma, lung cancer, renal cell carcinoma, Hodgkin's lymphoma, head and neck cancer, gastric cancer, mesothelioma, colorectal cancer, esophageal cancer, gastroesophageal junction cancer, hepatocellular carcinoma, glioblastoma, urothelial carcinoma, urinary bladder cancer, uterus cancer, primary central nervous system lymphoma, primary testicular lymphoma, biliary tract cancer, brain tumor, prostate cancer, leukemia, lymphoma, liver cancer, sarcoma, fibrosarcoma, mastocytoma, adrenocortical carcinoma, Ewing's tumor, multiple myeloma, testicular cancer, thyroid cancer, basal cell carcinoma, Paget's disease, or skin cancer.

15. A drug efficacy increasing agent of a medicament for treatment and/or prevention of cancer, wherein the agent comprises, as active ingredients, an anthracycline-based drug and a platinum formulation (but not comprising a pyrimidine-based drug as an active ingredient), wherein the medicament comprises, as an active ingredient, an antibody or a fragment thereof having an immunological reactivity with CAPRIN-1 protein.

16. The drug efficacy increasing agent according to claim 15, further comprising, as an active ingredient, an angiogenesis inhibitor.

17. A drug efficacy increasing agent of a medicament for treatment and/or prevention of cancer, wherein the agent comprises, as an active ingredient, an antibody or a fragment thereof having an immunological reactivity with CAPRIN-1 protein, wherein the medicament comprises, as active ingredients, an anthracycline-based drug and a platinum formulation (but not comprising a pyrimidine-based drug as an active ingredient).

18. The drug efficacy increasing agent according to claim 17, wherein the medicament further comprises, as an active ingredient, an angiogenesis inhibitor.

19. A method for treating and/or preventing cancer, comprising administering: an antibody or a fragment thereof having an immunological reactivity with CAPRIN-1 protein; an anthracycline-based drug; and a platinum formulation, together or separately to a subject (but not administering a pyrimidine-based drug).

20. The method according to claim 19, comprising further administering an angiogenesis inhibitor together or separately to a subject.
